(19)

Europäisches
Patentamt
European
Patent Office
Office européen
des brevets

(11) **EP 3 821 815 A1**

(12) **EUROPEAN PATENT APPLICATION**
published in accordance with Art. 153(4) EPC

(43) Date of publication:
**19.05.2021 Bulletin 2021/20**

(51) Int Cl.:
*A61B 10/00* (2006.01)   *G10L 25/66* (2013.01)

(21) Application number: **19835135.5**

(86) International application number:
**PCT/JP2019/027607**

(22) Date of filing: **11.07.2019**

(87) International publication number:
**WO 2020/013302 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **13.07.2018   JP 2018133356**
**25.03.2019   JP 2019057353**

(71) Applicants:
• **Life Science Institute, Inc.**
**Tokyo 100-8251 (JP)**

• **PST Inc.**
**Yokohama, Kanagawa 231-0023 (JP)**

(72) Inventors:
• **OMIYA, Yasuhiro**
**Yokohama, Kanagawa 231-0023 (JP)**
• **KUMAMOTO, Yorio**
**Tokyo 101-0047 (JP)**

(74) Representative: **Müller-Boré & Partner**
**Patentanwälte PartG mbB**
**Friedenheimer Brücke 21**
**80639 München (DE)**

(54) **MENTAL/NERVOUS SYSTEM DISORDER ESTIMATION SYSTEM, ESTIMATION PROGRAM, AND ESTIMATION METHOD**

(57)   There are provided an estimation system, an estimation program and an estimation method through which voice spoken by a subject is estimated, a disease from which the subject is suffering is distinguished and estimated, aggravation of the disease is prevented, and patients are able to receive appropriate treatment based on accurate distinguishing of the disease.

FIG. 1

EP 3 821 815 A1

**Description**

[Technical Field]

Cross-reference of related applications

Priority is claimed on Japanese Patent Application No. 2018-133356, filed July 13, 2018, and Japanese Patent Application No. 2019-057353, filed March 25, 2019, the content of which is incorporated herein by reference.

The present invention relates to an estimation system, an estimation program and an estimation method for psychiatric/neurological diseases.

[Background Art]

Compared to diseases (hereinafter referred to as "physical diseases") that impair physical health, there are many cases in diseases that impair mental health (hereinafter referred to as "psychiatric diseases") that the cause and mechanism of onset of the diseases have not been clarified. In addition, it is often difficult to perform a definitive diagnosis in which a part of a patient's body is collected for diagnosis and treatments thereof.

Regarding types of psychiatric diseases, for example, there is a disclosure of diagnostic criteria in "Diagnostic and Statistical Manual of Mental Disorders" (hereinafter referred to as "DSM-5") published by the American Psychiatric Association.

Here, "II. Schizophrenia Spectrum and Other Psychotic Disorders," "III. Bipolar Disorder and Related Disorders," "IV. Depressive Disorders," "V. Anxiety Disorders," "XVIII. Personality Disorders" and the like are generally recognized as psychiatric diseases. These psychiatric diseases affect a wide range of people, from young to old. In addition, these psychiatric diseases may impair daily life due to a decrease in motivation to be active resulting from depressed mood and a decrease in sociability such as difficulty communicating with others.

Particularly, suicide associated with severe depression such as major depressive disorder has also become a major problem. In addition, there is a large economic loss because of the working-age population suffering from such diseases not being able to work. The total cost for care of diseases including schizophrenia, depression, and anxiety disorders in Japan in 2008 was calculated to be about 6 trillion yen (Ministry of Health, Labour and Welfare: March 2011, "Estimation of social costs of psychiatric disorders" business report).

There is still prejudice against these psychiatric disorders for various reasons. Therefore, patients with these psychiatric disorders tend to hesitate to tell others that they are suffering from psychiatric disorders. Accordingly, there are problems that the detection and treatment of patients are delayed and as a result, no measures are taken until the condition becomes serious. In addition, the paucity of biomarkers effective for psychiatric disorders also contribute to delayed detection and treatment.

For example, for physical diseases, it is possible to detect physical diseases at an early stage by typical health examinations such as a blood test, an X-ray, an electrocardioagram and other various tests in many cases. On the other hand, for psychiatric diseases, it is difficult to predict a possibility of suffering from psychiatric diseases sufficiently because there are only a few questionnaire tests.

In addition, DSM-5 "XVII. Neurocognitive Disorders" are disorders related to so-called dementia, and the number of patients tends to increase with the aging of the population. At present, there is no curative drug for dementia such as Alzheimer's dementia and Lewy body dementia. Therefore, if symptoms progress, a social burden is caused, and a problem occurs because it particularly puts a heavy burden on the family. In response to this situation, the Ministry of Health, Labour and Welfare in Japan has announced a New Orange Plan (Comprehensive Strategy to Accelerate Dementia Measures) and is taking such measures.

In recent years, regarding a quantitative measurement method for observing the brain, there are an example in which a Single Photo Emission CT (SPECT) which measures a blood flow state in the brain is used for diagnosing dementia and Parkinson's disease and an example in which optical topography for measuring the concentration of hemoglobin in the blood in the cerebral cortex is used for diagnosing depression and the like. However, these measurement instruments are expensive and therefore, medical institutions in which such measurement can be performed are limited.

In recent years, a technique for measuring ethanolamine phosphate (EPA) in the blood and using it for diagnosing depression has been developed (refer to Patent Literature 3) and clinical tests are currently being conducted. In this test method, it has been indicated that it is possible to determine that the subject has depression because there is a significant correlation between "depression" and the concentration of ethanolamine phosphate in plasma. However, there is no method to distinguish between a plurality of disorders with depression symptoms such as depressive disorder, atypical depression, and bipolar disorder.

On the other hand, for example, it has been reported that, if depression can be detected in a prodromal stage or in a mild stage, various mental health services for recovering without medical treatment can be used (refer to Non-Patent Literature 1). Therefore, there is a need for a simple and patient-friendly test method for estimating psychiatric diseases.

In addition, in the related art, techniques for interfering an emotional or mental state (showing depression symptoms) by analyzing voice spoken by a person have been proposed (refer to Patent Literature 2 and 3). However, in this technique, it is not possible to estimate the type of disease.

[Citation List]

[Patent Literature]

[Patent Literature 1]
WO 2013/069645
[Patent Literature 2]
Japanese Unexamined Patent Application, First Publication No. 2007-296169
[Patent Literature 3]
WO 2006/132159

[Non Patent Literature]

[Non-Patent Literature 1]
Research Institute of Economy, Trade and Industry, Policy Discussion Paper Series 14-P-001 January 2004, "Proposal to Promote the Entry of the Private Sector into Non-clinical Depression Related Businesses as Providers of Low-intensity Mental Health Services"

[Summary of Invention]

[Technical Problem]

According to the diagnostic criteria of DSM-5, there are four subtypes of neurocognitive disorders (dementia): Alzheimer's dementia (Neurocognitive Disorder Due to Alzheimer's Disease), frontotemporal dementia (Frontotemporal Neurocognitive Disorder), Lewy body dementia (Neurocognitive Disorder with Lewy Bodies), and vascular dementia (Vascular Neurocognitive Disorder). In addition to these types, dementia caused by brain damage and other disorders has been demonstrated.

In addition, according to the diagnostic criteria of DSM-5, Parkinson's disease is included in the category of Lewy body dementia. Lewy body dementia and Parkinson's disease are distinguished between according to a region where Lewy bodies occur. Therefore, symptoms may be similar and difficult to distinguish.

Although these dementias cannot yet be completely cured, there are approved drugs that can delay the progression to some extent. However, since therapeutic agents differ depending on the type of dementia, it is necessary to estimate the type. In addition, advanced techniques are required for estimation. For example, it is so difficult to estimate the type of dementia that it is generally said that Alzheimer's dementia can only be initially definitively diagnosed after death by examining the brain of a patient.

Therefore, if the types of dementia could be distinguished between in a state in which the degree of dementia was mild, that is, in a state of mild cognitive impairment (MCI), this state could be maintained for a longer time.

In addition, regarding psychiatric diseases with mood swings such as schizophrenia, major depressive disorder, depressive disorder with atypical features (hereinafter referred to as "atypical depression"), bipolar disorder, generalized anxiety disorders, personality disorders, persistent depressive disorder, and cyclothymic disorders, when the degree of symptoms is mild, the patient's disorder tends to be regarded as a problem other than the above psychiatric diseases, for example, regarded as simply a personality problem, simply being lazy, or simply due to poor physical condition, and the patient himself or herself may be unaware of his or her disease.

In addition, for example, even if a patient becomes aware of a disorder, the patient may not receive medical care due to prejudice against psychiatric diseases and a feeling of resistance to visiting a psychiatric and psychosomatic medical department. In addition, depending on the country or region, due to social issues such as a small number of specialized counselors and customs that it is not common to consult a specialized counselor, social services as a method for recovering from a disorder may not be sufficiently utilized. Therefore, even if a patient becomes aware of a disorder at an early stage, the disorder may become chronic and the symptoms may become severe.

In addition, since psychiatric diseases have common symptoms, it may be difficult to identify the disorders. For example, it may be difficult to distinguish between Alzheimer's dementia and frontotemporal dementia, between Alzheimer's dementia and Lewy body dementia, between Lewy body dementia and Parkinson's disease, and between bipolar disorder and major depressive disorder.

In addition, patients may have dementia and senile depression. In addition, patients may exhibit depression symptoms as early symptoms of dementia in many cases. On the other hand, when patients have depressive pseudo-dementia, the patients are actually depressed, but symptoms such as declined cognitive functions appear. Therefore, it is an important task to distinguish between whether the patient has a type of dementia, a type of depression, or a combination of dementia and depression in which one symptom is exhibited significantly. This is because, for example, when a patient has depressive pseudo-dementia, cognitive functions are recovered if the patient is treated for depression, but if the patient is treated for dementia, the patient does not recover or the symptoms worsen.

The present invention has been made in view of the above circumstances, and an object of the present invention is to provide an estimation, an estimation system, an estimation program and an estimation method for psychiatric/neurological diseases through which it is possible to distinguish and estimate dementia by analyzing voice spoken by a subject.

In addition, another object of the present invention is to provide an estimation system, an estimation program and an estimation method for psychiatric/neurological diseases through which it is possible to distinguish between and estimate psychiatric diseases with mood swings such as bipolar disorder and major depressive disorder by analyzing the voice spoken by a subject.

In addition, still another object of the present invention is to provide an estimation system, an estimation program and an estimation method for psychiatric/neurological diseases through which it is possible to distinguish between psychiatric diseases having very similar symptoms, reduce misdiagnosis, perform early and accurate distinguishing thereof, and perform appropriate treatments.

In addition, still another object of the present invention is to provide an estimation system, an estimation program and an estimation method for psychiatric/neurological diseases with which an operation is simple, measurement is possible without requiring a special environment, the burden on patients is small, and psychiatric/neurological diseases can be estimated by measurement in a short time.

In addition, still another object of the present invention is to provide an estimation system, an estimation program and an estimation method for psychiatric/neurological diseases through which, even if patients and doctors are physically separated from each other, measurement is possible, and psychiatric/neurological diseases can be estimated.

[Solution to Problem]

In order to address the above problems, the present invention provides an estimation system for psychiatric/neurological diseases including at least one voice input part including an acquisition part configured to acquire voice data of a subject and a first transmitting part configured to transmit the voice data to a server; a result display part including a first receiving part configured to receive data of the result estimated in the server and an output part configured to display the data; and a server including a second receiving part configured to receive the voice data from the first transmitting part, a calculating part configured to calculate a predictive value of a disease based on a combination of at least one acoustic feature value extracted from the voice data of the subject, an estimation part configured to estimate the disease of the subject, and a second transmitting part configured to transmit the estimated data of the result to the display part, wherein the server includes an arithmetic processing device for executing an estimation program and a recording device in which the estimation program is recorded, and the estimation program enables the estimation part to estimate the disease of the subject selected from the group consisting of a plurality of disorders using the predictive value of the disease as an input.

[Advantageous Effects of Invention]

According to the present invention, it is possible to provide an estimation system, an estimation program and an estimation method through which voice spoken by a subject is estimated, a disease from which the subject is suffering is distinguished and estimated, aggravation of the disease is prevented, and patients are able to receive appropriate treatment based on accurate distinguishing of the disease.

[Brief Description of Drawings]

Fig. 1 is a configuration diagram of the present invention.
Fig. 2 is a configuration diagram of the present invention.

Fig. 3 is a flowchart of the present invention.

Fig. 4A is a diagram showing an example of scoring.

Fig. 4B is a conceptual diagram showing a disease-specific distribution of predictive values of disease.

Fig. 5A is an ROC curve showing accuracy of estimation of the present invention.

Fig. 5B is an ROC curve showing accuracy of estimation of the present invention.

Fig. 6 is a diagram explaining a second acoustic parameter.

Fig. 7 is a diagram of regression analysis of the present invention.

Fig. 8 is a diagram of regression analysis of the present invention.

Fig. 9 is a diagram showing an example of a distribution of zero crossing rates ZCR and Hurst exponents H of the present invention.

Fig. 10 is a diagram showing a Parkinson's disease index of the present invention.

Fig. 11 is a diagram showing plotted voice data of diseases of the present invention.

[Description of Embodiments]

(Embodiments)

Forms for implementing the present invention will be described below with reference to the drawings.

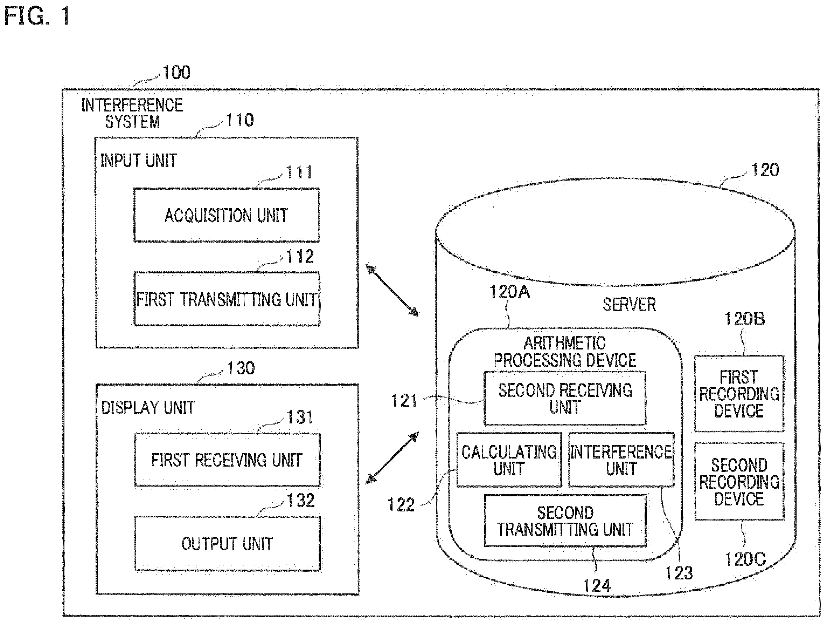

Fig. 1 is a configuration diagram of an estimation system 100 of the present invention. The estimation system 100 in Fig. 1 includes an input part 110 for acquiring a subject's voice, a display part 130 for displaying estimation results and the like for a subject, and a server 120. The server 120 includes an arithmetic processing device 120A (CPU), a first recording device such as a hard disk in which an estimation program for the arithmetic processing device 120A to execute processes is recorded, and a second recording device such as a hard disk in which medical examination data of the subject and a collection of messages to be sent to the subject are recorded. The server 120 is connected to the input part 110 and the display part 130 via a wired or wireless manner. The arithmetic processing device 120A may be realized by hardware.

The input part 110 includes a voice acquisition part 111 such as microphone and a first transmitting part 112 configured to transmit the acquired voice data to the server 120. The acquisition part 111 generates voice data of a digital signal from an analog signal of the subject's voice. The voice data is transmitted from the first transmitting part 112 to the server 120.

The input part 110 acquires a signal of voice spoken by the subject through the voice acquisition part 111 such as a microphone and samples the voice signal at a predetermined sampling frequency (for example, 11,025 Hz) to generate voice data of a digital signal.

The input part 110 may include a recording part configured to record voice data separately from the recording device on the side of the server 120. In this case, the input part 110 may be a portable recorder. The recording part may be a recording medium such as a CD, a DVD, a USB memory, an SD card, or a minidisk.

The display part 130 includes a first receiving part 131 configured to receive data such as estimation results and an output part 132 configured to display the data. The output part 132 is a display that displays data such as estimation results. The display may be an organic electro-luminescence (EL) display, a liquid crystal display, or the like.

Here, the input part 110 may have a function of a touch panel in order to input data of results of the health examination to be described below and data of answers regarding stress check in advance. In this case, it may be realized by the same hardware having functions of the input part 110 and the display part 130.

The arithmetic processing device 120A includes a second receiving part 121 configured to receive voice data transmitted from the first transmitting part 112, a calculating part 122 configured to calculate a predictive value of a disease based on the acoustic feature value extracted from the voice data of a subject, an estimation part 123 configured to estimate a disease of a subject using an estimation model learned by machine learning using the predictive value of the disease as an input, and a second transmitting part 124 configured to transmit data regarding the estimation result and the like to the display part 130. Here, while the calculating part 122 and the estimation part 123 are described separately for explaining functions, the functions of the calculating part and the estimation part may be performed at the same time. Here, in this specification, the term "mental value" is used synonymously with the predictive value of the disease.

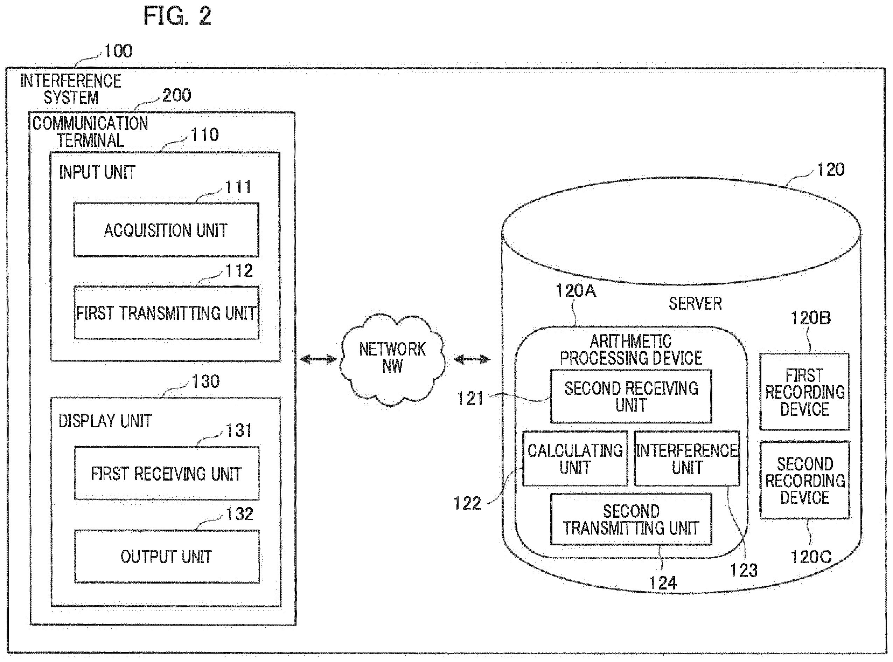

Fig. 2 shows another configuration diagram of the estimation system 100 of the present invention. The estimation system 100 in Fig. 2 connects the server 120 of the estimation system 100 in Fig. 1 to the input part 110 or the display part 130 via a network NW. In this case, the input part 110 and the display part 130 are a communication terminal 200. The communication terminal 200 is, for example, a smartphone, a tablet terminal, or a laptop or desktop computer including a microphone.

The network NW connects the communication terminal 200 to the server 120 via a mobile phone communication network or a wireless LAN based on communication standards such as Wi-Fi (Wireless Fidelity) (registered trademark).

The estimation system 100 may connect a plurality of communication terminals 200 to the server 120 via the network NW

The estimation system 100 may be realized by the communication terminal 200. In this case, an estimation program stored in the server 120 is downloaded via the network NW and recorded in the recording device of the communication terminal 200. When a CPU included in the communication terminal 200 executes an application recorded in the recording device of the communication terminal 200, the communication terminal 200 may function as the calculating part 122 and the estimation part 123. The estimation program may be distributed by being recorded on an optical disc such as a DVD or a portable recording medium such as a USB memory.

(First embodiment)

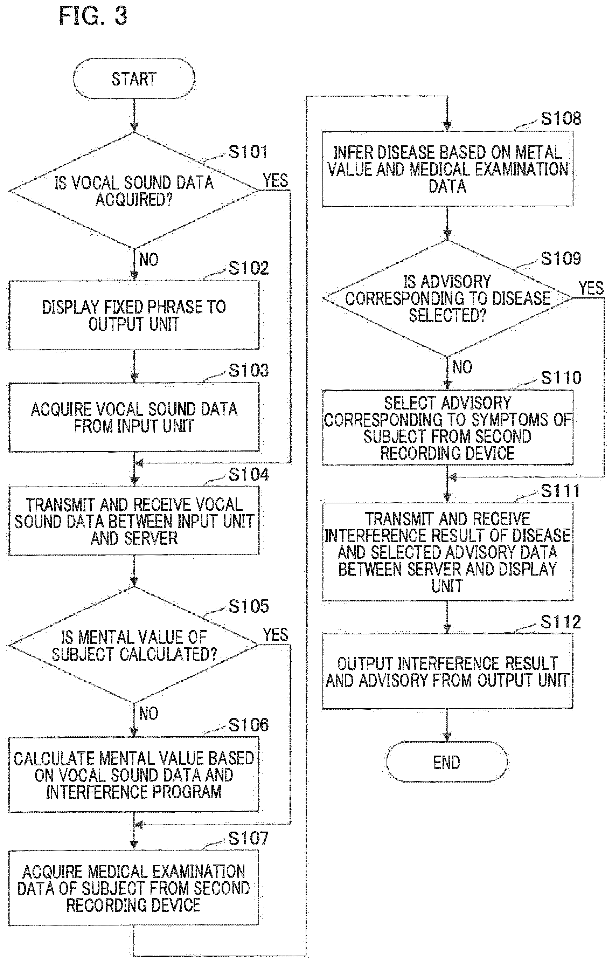

Fig. 3 shows an example of an estimation process of the estimation system 100 shown in Fig. 1. The process shown in Fig. 3 is realized when the arithmetic processing device 120A of the estimation system 100 executes an estimation program recorded in a first recording device 120B of the estimation system 100. Functions of the second receiving part 121, the calculating part 122, the estimation part 123 and the second transmitting part 124 of the arithmetic processing device 120A will be described with reference to Fig. 3.

(Calculating part 122)

When the process starts, in Step S101, the calculating part 122 determines whether voice data has been acquired by the acquisition part 111. If the voice data has already been acquired, the process proceeds to Step S104. If the voice data has not been acquired, in Step S102, the calculating part 122 instructs the output part 132 of the display part 130 to display a predetermined fixed phrase.

This estimation program does not estimate psychiatric/neurological diseases based on the meaning or content of the subject's utterance. Therefore, the acquired voice data may be any data as long as the utterance time is 15 seconds to 300 seconds. The language used is not particularly limited, but it is desirable to match the language used by the population when the estimation program is created. Therefore, the fixed phrase displayed on the output part 132 may be any fixed phrase as long as it is the same language as the population and has an utterance time of 15 seconds to 300 seconds. Preferably, a fixed phrase having an utterance time of 20 seconds to 180 seconds is desirable.

For example, the phrase may be "I-ro-ha-ni-ho-he-to" or "A-i-u-e-o-ka-ki-ku-ke-ko" that does not contain specific emotions or may be a question such as "What is your name?" or "When is your birthday?"

The voice acquisition environment is not particularly limited as long as it is an environment in which only voice spoken by the subject can be acquired, and it is preferable to acquire voice in an environment of 40bB or less. Preferably, it is preferable to acquire voice in an environment of 30 dB or less.

When the subject reads out the fixed phrase, in Step S103, voice data is acquired from the voice spoken by the subject, and the process proceeds to Step S104.

Next, in Step S104, the calculating part 122 instructs the voice data to be transmitted from the input part 110 to the second receiving part 121 of the server 120 through the first transmitting part 112.

Next, in Step S105, the calculating part 122 determines whether the predictive value of the disease of the subject has been calculated. The predictive value of the disease is a feature value F(a) obtained by a combination of acoustic feature values generated by extracting one or more acoustic parameters and is a predictive value of a specific disease used as an input of an estimation algorithm for machine learning. The acoustic parameters are parameters of features during sound transmission. When the predictive value of the disease has already been calculated, the process proceeds to Step S107. When the predictive value of the disease has not been calculated, in Step S106, the calculating part 122 calculates the predictive value of the disease based on the voice data of the subject and the learned estimation program.

Here, the following Step S107 to Step S112 will be described in detail in the following paragraph, but will be briefly described here. In Step S107, the calculating part 122 acquires medical examination data of the subject acquired in advance from a second recording device 120C. Here, Step S107 may be omitted and the disease may be estimated from the predictive value of the disease without acquiring medical examination data.

Next, in Step S108, the estimation part 123 estimates the disease based on the predictive value of the disease calculated by the calculating part 122 and/or the medical examination data.

For the predictive value of the disease, when individual threshold values for distinguishing a specific disease from others are set, a plurality of patients for whom the predictive value of the disease is calculated can be distinguished as subjects to be specified or others. In the embodiment to be described below, determination is made by classifying a case in which the threshold value is exceeded and a case in which the threshold value is not exceeded.

Next, in Step S109, the estimation part 123 determines whether advisory data corresponding to the disease has been selected. The advisory data corresponding to the disease is an advice for preventing the disease or avoiding aggravating of the disease when the subject receives the advisory data. If the advisory data has been selected, the process proceeds to Step S111.

If the advisory data has not been selected, in Step S110, advisory data corresponding to the symptoms of the subject is selected from the second recording device.

Next, in Step Sill, the estimation part 123 instructs the estimation result of the disease and the selected advisory data to be transmitted to the first receiving part 131 of the display part 130 through the second transmitting part 124.

Next, in Step S112, the estimation part 123 instructs the output part 132 of the display part 130 to output the estimation result and the advisory data. Finally, the estimation system 100 ends the estimation process. (Second embodiment)

Since the estimation program of the present invention can also analyze voice from a remote place, it can be used in online medical treatment and online counseling. When psychiatric/neurological diseases are diagnosed, doctors observe a patient's facial expression, movement, conversation status, and the like through inquiries or interviews. However, since patients may have prejudice against psychiatric/neurological diseases, they may hesitate to go to psychiatric hospitals or clinics.

Online medical treatment and counseling can be performed by interviewing of doctors and counselors without visiting a facility. Therefore, compared to diseases other than psychiatric/neurological diseases, the psychiatric/neurological diseases have a very high affinity for online medical treatment.

Doctors, counselors, and clinical psychologists can perform analysis with this estimation program during online interviews with patients (or clients). Therefore, this makes it very easy to estimate whether a person is affected with a psychiatric/neurological system disease or the type of the disease. Here, during interview, various psychology tests and cognitive function tests such as MMSE, BDI, and PHQ-9 can be performed together.

In this case, patients need to have a monitor screen for interviewing and a microphone for voice recording in addition to computer hardware that can transmit voice.

If patients do not have these devices at home, the devices can be provided in, for example, family clinics. Patients can go to their family clinics and can be interviewed through the devices there.

In addition, for example, when a patient visits a family clinic in order to treat a physical disease, if a family doctor diagnoses and determines that there is a suspicion of psychiatric/neurological diseases, voice can be acquired in the clinic and analyzed by the program of the present invention.

In other places also, if psychiatrist and neurologists can perform online medical treatment, the family doctor, psychiatrist, and neurologist can perform diagnosis through online collaboration with each other.

(Third embodiment)

The estimation program of the present invention can be used as a screening tool by increasing the sensitivity for estimating a specific disease (in this case, the specificity generally decreases).

When the program is used as an inspection item for health examinations conducted in companies and local governments and clinical surveys conducted in medical institutions, it can contribute to detecting psychiatric and neurological disorders at an early stage in which they have been difficult to detect so far and for which there is no simple test method.

For example, as in a fundus test, a visual acuity test, and an audibility test, it is possible to acquire voice as one of a series of tests and notify of the estimation result by the program in that place or together with other test results.

(Fourth embodiment)

Since the estimation program of the present invention does not require a special device, it can be easily used by anyone. On the other hand, since its application is limited to psychiatric/neurological diseases, the application frequency is not always high. Therefore, a set of estimation devices of the present invention is provided in a specialized hospital in which an expensive test device is provided, and a family doctor or the like can request a test from the specialized hospital when a target patient visits.

Examples of devices used for psychiatric/neurological diseases include optical topography, myocardial scintigraphy, cerebral blood flow scintigraphy, CT, MRI, and electroencephalography. These are used for disease estimation and exclusive diagnose, but the estimation device of the present invention has very low invasiveness, it can be used in combination with these tests or before these tests.

(Fifth embodiment)

Since the estimation program of the present invention can be easily used at home, it can be used as a monitoring device after diagnosis. For example, in the case of mood disorder diseases, drugs and psychotherapies are provided according to the patient's disease, and the effectiveness of these therapies can be measured. In addition, when the program is continuously used, it is possible to observe daily whether the symptom is stable and whether there is a sign of recurrence.

(Sixth embodiment)

Since the estimation program of the present invention analyzes voice produced by utterance, it can be applied as a monitoring device for elderly people.

Whether an elderly person who lives alone is well is a matter of concern to his or her close relatives. When the estimation program of the present invention is implemented in an elderly person monitoring system using a communication device such as a phone or a video phone, it is possible not only to observe the life reaction but also to measure whether there is a tendency for dementia or depression, and it is possible to take appropriate measures for people living alone.

In these various embodiments, the voice acquisition method is not particularly limited, and examples thereof include (1) a method in which recorded voice is sent from an examinee via a phone or the Internet, (2) a method in which an inspector makes contact with an examinee via a phone or the Internet and acquires voice from a conversation, (3) a method in which a voice acquisition device is installed in a house of an examinee and the examinee records voice in the device, and (4) a method in which a voice acquisition device automatically starts regularly and acquires voice of an examinee through a conversation with the examinee.

In acquisition of voice, it is preferable to display sentence to be spoken on a display of the estimation device or to reproduce sound of sentence to be spoken from a speaker so that the examinee can speak smoothly.

Recording starts according to mechanical sound indicating recording start and when the utterance is completed, recording is ended with a switch, and voice of the utterance for each sentence can be acquired

(Calculation of predictive value of disease)

The outline of calculation of the predictive value of diseases in the present invention will be described. As preprocessing for the estimation part 123 in Fig. 1 to perform the estimation process, the calculating part 122 undergoes a step of extracting a plurality of acoustic parameters from voice data of the subject. Acoustic parameters are extracted from patient's voice data. Acoustic parameters are parameters of characteristics of sound transmission. Examples of acoustic parameters include a zero crossing rate and a Hurst exponent. The zero crossing rate is obtained by calculating the number of times in a part time that the waveform of a sound pressure of voice crosses a reference pressure as a degree of intensity in change of the waveform in voice. The Hurst exponent indicates a correlation of changes in the waveform of voice.

The acoustic parameter includes a first acoustic parameter and a second acoustic parameter. The first acoustic parameter is an acoustic parameter extracted from voice of a patient whose specific disease should be estimated. The second acoustic parameter is an acoustic parameter that is recorded in a database 120B in advance. The second acoustic parameter is extracted from voice data of patients with Alzheimer's dementia, Lewy body dementia, Parkinson's disease, major depressive disorder, atypical depression, or bipolar disorder, and each acoustic parameter and each disease are linked in advance.

The acoustic parameters used in the present invention include the following items.

1) Envelope of volume (attack time, decay time, sustain level, and release time)
2) Waveform variation information (Shimmer, Jitter)
3) Zero crossing rate
4) Hurst exponent
5) Voice Onset Time (VOT)
6) Statistical values of inter-speech distribution related to Mel Frequency Cepstral coefficients (the first quartile, the median value, the third quartile, the 95th percentile, the arithmetic mean, the geometric mean, a difference between the third quartile and the median value, etc.)
7) Statistical values of inter-speech distribution at the speed of change in frequency spectrum (the first quartile, the median value, the third quartile, the 95th percentile, the arithmetic mean, the geometric mean, a difference between the third quartile and the median value, etc.)
8) Statistical values of inter-speech distribution related to time change of Mel Frequency Cepstral coefficients (the first quartile, the median value, the third quartile, the 95th percentile, the arithmetic mean, the geometric mean, a difference between the third quartile and the median value, etc.)
9) Statistical values of inter-speech distribution related to time change of Mel Frequency Cepstral coefficients (the first quartile, the median value, the third quartile, the 95th percentile, the arithmetic mean, the geometric mean, a difference between the third quartile and the median value, etc.)
10) Square error for quadratic regression approximation in change of utterance with 90% roll-off frequency spectrum over time

11) Arithmetic error for quadratic regression approximation in time variation in utterance with center of gravity of frequency spectrum

In addition, a pitch rate, a probability of voice, a power of frequency in an arbitrary range, a scale, a speaking speed (the number of mora in a certain time), pause, a volume and the like may be exemplified.

The estimation program has a learning function using artificial intelligence and performs the estimation process according to the learning function. Neural network type deep learning may be used, and reinforcement learning in which a learning field is partially reinforced and the like may be used, and other genetic algorithms, cluster analyses, self-organizing maps, ensemble learning, and the like may be used. Of course, other techniques related to artificial intelligence may be used. In ensemble learning, a classification algorithm may be created by a method using both boosting and a decision tree.

In the step of creating an estimation program, the algorithm creator examines any acoustic parameter used as a variable f(n) from among the second acoustic parameter items to obtain a better combination by a stepwise method and selects one or more thereof. Next, a coefficient is added to any selected acoustic parameter to create one or more acoustic feature values. In addition, these acoustic feature values are combined to create a feature value F(a).

There are three types of stepwise method: a variable increase method, a variable decrease method, and a variable increase and decrease method, but any of them may be used. Regression analysis used in the stepwise method includes a process of linear classification such as linear discriminant analysis and logistic regression analysis. The variable f(n) and coefficients thereof, that is, the coefficient xn in Formula F(a) represented by the following formula is called a regression coefficient, and is a weight applied to the function f(n).

After the regression coefficient is selected by the creator of the learning algorithm, the quality may be improved by machine learning for increasing estimation accuracy from disease information accumulated in the database.

The predictive value of the disease of the subject is calculated from one or more acoustic feature values based on, for example, the following formula F(a).

[Math. 1]

$$F(a) = x1 \times f(1) + x2 \times f(2) + x3 \times f(3) + \cdots + xn \times f(n)$$

Here, in f(n), any one or more second acoustic parameters are arbitrarily selected from among the above acoustic parameter items (1) to (11). xn is a disease-specific regression coefficient. f(n) and xn may be recorded in a recording device 120 of the estimation program in advance. The regression coefficient of the feature value F(a) may be improved in the process of machine learning of the estimation program.

The calculating part 122 in Fig. 1 calculates a feature value for distinguishing between healthy subjects and subjects with diseases or distinguishing between diseases based on a combination of the second acoustic parameters. From the feature value, the predictive value of the disease of the subject is calculated by scoring to calculate how far the value of the subject is from the reference range.

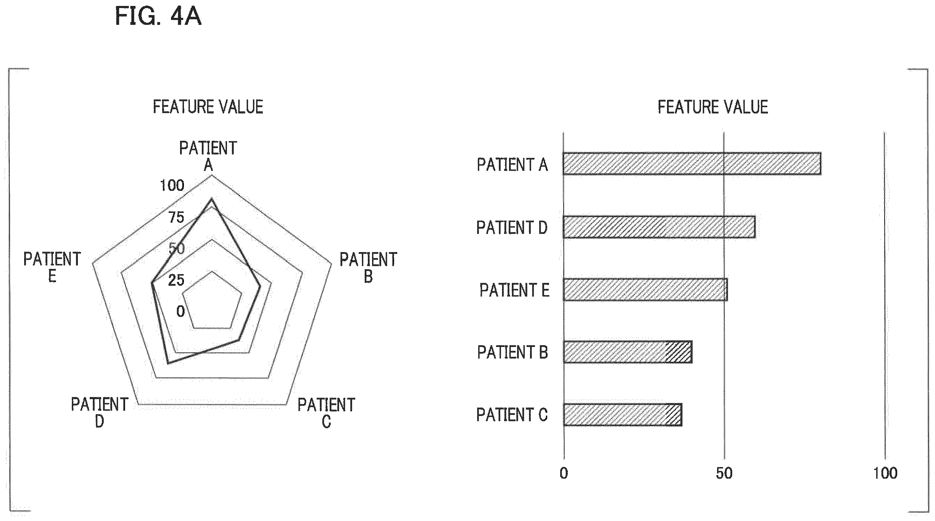

Fig. 4A is an image diagram showing the intensity of one acoustic feature value that differs for each disease. The subject with the disease A has the highest score. Therefore, the predictive value calculated for the disease A of the subject is higher than that of other disease groups. In addition, for example, when an intensity of 50 is set as a threshold value, diseases can be classified into a group of the disease A, the disease B, and the disease C and a group of the disease D and the disease E.

In Fig. 4A, the predictive value of the disease is calculated based on the intensity of one acoustic feature value, but in reality, it is difficult to classify the disease only with one acoustic feature value. Therefore, the feature value F(a) obtained by combining some acoustic feature values may be calculated to classify the disease.

Based on the feature value F(a), the predictive value of the disease is calculated for the voice of the labeled subject, and the distribution of predictive values of each disease is obtained. Thereby, each disease can be classified.

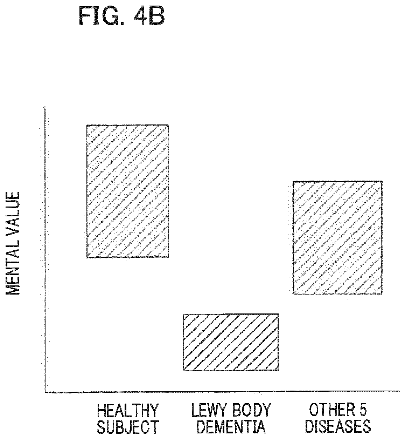

Fig. 4B is a distribution image showing the predictive value (described as a "mental value" in Fig. 4B) of the disease obtained by a combination of three acoustic parameters.

In Fig. 4B, it can be understood that the distribution of predictive values of a patient group with Lewy body dementia can be separated from the distribution of predictive values of a patient group with other diseases and a healthy subject group. In the present invention, a combination of acoustic parameters is set for each disease so that it can be distinguished from other diseases, a feature value F(a) is calculated, and it is possible to determine a disease to which voice of each examinee corresponds using the feature value F(a) as an input.

As another method, the feature value F(a) for each disease is extracted from voice of each patient, a disease of which the feature value is larger is determined, predictive values of diseases are compared, and thus it is possible to estimate a disease which the patient is suffering from.

In this case, the predictive value of the disease can be regarded as the degree of suffering from the disease. When the predictive values of the diseases are compared and recalculated, it is possible to express which disease the patient is suffering from with a probability.

In this manner, from voices of patients with six diseases including Alzheimer's dementia, Lewy body dementia, Parkinson's disease, major depressive disorder, atypical depression and bipolar disorder, and voices of healthy subjects, the feature value (a) associated with each disease is extracted and the predictive value of each disease is calculated.

In addition, regarding target diseases, the estimation program may be created from voices of patients including ten diseases additionally including the four diseases of vascular dementia, frontotemporal dementia, cyclothymia, and persistent depressive disorder.

Specifically, when voice spoken by a distinguishing target person is analyzed, it is estimated whether the voice corresponds to any of the above six diseases to ten diseases or whether the person is healthy.

(Estimation flow of estimation program)

Regarding the estimation flow of the estimation program, as described above, for each disease, the feature value (a) of each disease may be extracted and the predictive value of the disease may be calculated. However, first, a combination of acoustic feature values related to the disease group is created, the feature value F(a) related to the disease group is used as an input to the estimation part, inputting and estimation are performed in a plurality of steps, and thus each disease or wellness may be finally estimated.

(Estimation flow 1)

For example, first, in the first step, subjects are estimated to belong to one of three groups including (1-A) a dementia group including Alzheimer's dementia, Lewy body dementia, and Parkinson's disease, (1-B) a mood disorder group including major depressive disorder, atypical depression, and bipolar disorder, and (1-C) a healthy group.

Next, in the second step, according to the program that estimates whether voices of patients classified as the (1-A) dementia group correspond to any of three diseases including (1-A-) Alzheimer's dementia, (1-A-2) Lewy body dementia, and (1-A-3) Parkinson's disease, the diseases of the patients in the dementia group are estimated. On the other hand, according to the program that estimates whether voices of patients classified as the mood disorder group (1-B) correspond to any of three diseases including (1-B-1) major depressive disorder, (1-B-2) atypical depression, and (1-B-3) bipolar disorder, the diseases of the patients in the mood disorder group are estimated.

(Estimation flow 2)

As another aspect of the determination flow, first, in the first step, subjects are estimated to belong to one of three groups including (2-A) a dementia group including Alzheimer's dementia, Lewy body dementia, and Parkinson's disease, (2-B) a mood disorder group including major depressive disorder, atypical depression, and bipolar disorder, and (2-C) a healthy group.

Next, in the second step, it is determined whether the disease is Lewy body dementia according to a program that estimates whether (2-A) voices of patients classified as the dementia group correspond to (2-A-1) Lewy body dementia or other dementias. On the other hand, it is determined whether the disease is major depressive disorder according to a program that estimates whether (2-B) voices of patients classified as the mood disorder group correspond to (2-B-1) major depressive disorder or other mood disorder.

Similarly, using a program that estimates whether voices correspond to (2-A-2) Alzheimer's dementia or other dementias, a program that estimates whether voices correspond to (2-A-3) Parkinson's disease or other dementias, a program that estimates whether voices correspond to (2-B-2) atypical depression or other mood disorder, and a program that estimates whether voices correspond to (2-B-3) bipolar disorder or other mood disorder, finally, it is possible to determine whether the disease is any of Alzheimer's dementia, Lewy body dementia, Parkinson's disease, major depressive disorder, atypical depression and bipolar disorder.

(Estimation flow 3)

In addition, another embodiment, in the second step, for (3-A) voices of the patients classified as the dementia group, a combination of three estimation programs including (3-A-1) a program that estimates whether the voice corresponds to Alzheimer's dementia or Lewy body dementia, (3-A-2) a program that estimates whether the voice corresponds to Lewy body dementia or Parkinson's disease, and (3-A-3) a program that estimates whether the voice corresponds to Parkinson's disease or Alzheimer's dementia is used, and thus the diseases of the patients classified as the dementia group are estimated.

On the other hand, for (3-B) voices of the patients classified as the mood disorder group, a combination of three estimation programs including (3-B-1) a program that estimates whether the voice corresponds to major depressive disorder or atypical depression, (3-B-2) a program that estimates whether the voice corresponds to atypical depression or bipolar disorder, and (3-B-3) a program that estimates whether the voice corresponds to bipolar disorder or major depressive disorder is used, and thus the disease of the patients classified as the mood disorder group is estimated.

(Estimation flow 4)

In addition, the first step in which subjects are divided into three classes including the above dementia group, the mood disorder group, and the healthy group may be two steps in which subjects are first divided into two classes including (4-A) a healthy group and (4-B) another disease group, and then the disease group is divided into (4-B-1) a dementia group and (4-B-2) a mood disorder group.

(Examinee of voice acquired when estimation program is created)

The acquisition of voice data of the second acoustic parameter will be described. It is preferable to select an examinee whose voice will be acquired based on the following criteria.

(A) Examinees will give written consent after receiving sufficient explanation that the acquired voice will be used for disease analysis.

(B) This estimation system does not perform estimation analysis of diseases based on the meaning or content (text) of words. Therefore, there are basically no limitations on nationality or first language. However, since there may be differences between races and between languages, it is preferable to determine the same race and language as comparison subjects. For example, in consideration of convenience, when the present invention is performed in Japan, it is preferable to acquire voice from people whose first language is Japanese to create this estimation program and estimate diseases by Japanese utterance. In addition, when the present invention is performed in an English-speaking country, it is preferable to acquire voice from people whose first language is English to create this estimation program and estimates diseases by English utterance.

(C) Age is not particularly limited as long as the subject can speak. However, in consideration of voice change and emotional stability, an age of 15 or more is preferable, an age of 18 or more is more preferable, an age of 20 or more is particularly preferable, and in consideration of difficulty in physical utterance due to age, an age of less than 100 is preferable and an age of less than 90 is more preferable.

(D) When the present invention is performed in Japan, people who can read (speak) Japanese sentences when voice is acquired are preferable. However, when people whose first language is not Japanese are used for estimation, it is possible to perform determination when they read sentences of their first language.

(E) When the estimation algorithm is created, it is preferable to use voices of people who have each of six diseases including Alzheimer's dementia, Lewy body dementia, Parkinson's disease, major depressive disorder, atypical depression and bipolar disorder. However, people who have these diseases are excluded. In addition to these six diseases, it is possible to use voices of people who have each of vascular dementia, frontotemporal dementia, persistent depressive disorder, and cyclothymic disorders. In addition, it is possible to use voices of people who have each of schizophrenia, generalized anxiety disorders, and other psychiatric diseases

(F) Healthy subjects are preferably people who have been confirmed to have neither dementia nor mood disorders.

(Device for acquiring voice)

A device for acquiring voice will be described.

(1) The microphone is not particularly limited as long as it can acquire voice. For example, a handheld microphone, a headset, a microphone built into a mobile terminal, or a microphone built into a personal computer, a tablet, or the like may be selected. A pin microphone, a directional microphone, or a microphone built into a mobile terminal is preferable because only voice of an examinee can be acquired.

(2) For the recorder, a recording medium that is built into or externally added to a portable recorder, a personal computer, a tablet, or a mobile terminal can be used.

(3) There are no limitations on utterance content when diseases are estimated. For example, utterances freely produced by examinees, utterances produced when examinees read a sentence prepared in advance, and utterances produced by phone or face-to-face conversation can be used. However, when the estimation algorithm is created, it is preferable to use utterance content common to the examinees. Therefore, when the estimation algorithm is created, it is preferable that the examinees read and speak a sentence prepared in advance.

If the utterance time is too short, the accuracy of the estimation result decreases. The utterance time is preferably 15 seconds or longer, more preferably 20 seconds or longer, and particularly preferably 30 seconds or longer. In addition, if the utterance time is longer than necessary, obtaining the results takes time. The utterance time is preferably 5 minutes or shorter, more preferably 3 minutes or shorter, and particularly preferably 2 minutes or shorter.

(4) Observation and inspection items are not particularly limited. However, it is preferable to obtain information in order to verify whether there is a difference in voice due to the difference in the examinee's profiles. Information includes a gender, an age, a height, a weight and the like as general information, includes a definitive diagnosis name, severity, complications of physical diseases, a medical history, a time of onset, and the like as medical information, includes MRI, CT, and the like as inspection information, and includes Patient Health Questionnaire (PHQ)-9, The M.I.N.I-International Neuropsychiatric Interview (M. I. N. I. screen), Hamilton Depression Rating Scale (HAM-D or HDRS), Young Mania Rating Scale (YMRS), Mini-Mental State Examination (MMSE), Bipolar Spectrum Diagnostic Scale (BSDS), The Unified Parkinson's Disease Rating Scale (MDS-UPDRS) revised by The Movement Disorder Society, and the like as inquiries and questions.

(5) Regarding an environment in which voice is acquired, there is no particular limitation as long as it is an environment in which only the patient's utterance can be acquired. A quiet environment, specifically, an environment of 40 dB or less is preferable, and an environment of 30 dB or less is more preferable. Specific examples thereof include an examination room, a counseling room, a conference room, an audibility test room, a CT, MRI, or X-ray test room. In addition, voice may be acquired in a quiet room of the examinee's home.

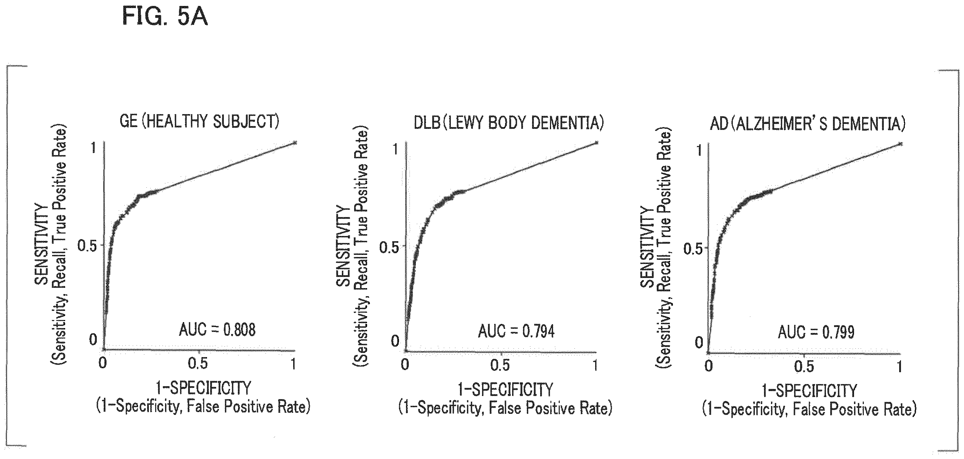

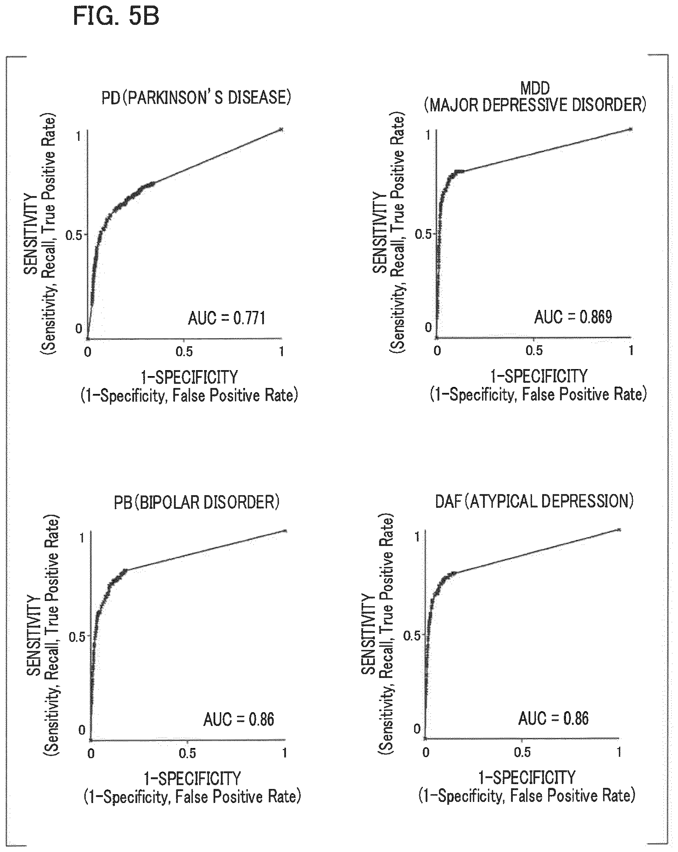

Fig. 5 shows graphs of ROC curves showing healthy subjects or specific diseases, and other separation performances. The horizontal axis represents 1-specificity, and the vertical axis represents sensitivity. In other words, the horizontal axis represents false positive rate and the vertical axis represents true positive rate. The ROC curves in Fig. 5 always show values with a high true positive rate when the false positive rate is low.

In addition, the area under the ROC curve (AUC) was high in all cases, and a significant difference was confirmed in the case of random identification. Diseases for which separation performance has been verified are Lewy body dementia, Parkinson's disease, and major depressive disorder. The AUC in each ROC curve was 0.794 for Lewy body dementia, 0.771 for Parkinson's disease, and 0.869 for major depressive disorder. Here, diseases that can be estimated using the present invention is not limited to the above examples. Based on the results of AUC, it can be confirmed that the estimation accuracy of the disease is high.

The estimation program created as described above can be used for those suspected of having psychiatric diseases and those who are presumed to be healthy without particular limitation. In a usage scenario, it can be easily used as a tool for medical examination by a doctor or an inspection item for a health examination or a clinical survey as long as the voice of the examinee is acquired.

Regarding the number of times of voice acquisitions, one voice acquisition can be used for distinguishing. However, for example, even healthy people may exhibit a depressed state due to life events and like, and their moods may be depressed in many cases when voice is acquired. In addition, regarding major depressive disorder, atypical depression, and the like, good and bad moods may change between morning and night. Therefore, when the estimation result indicates that the subject is suffering from some kind of psychiatric disorder, it is preferable to perform voice acquisition again at least once and perform estimation again.

(Estimation of diseases by combining elements other than voice)

Estimation of diseases by combining elements other than voice will be described. The estimation system for psychiatric/neurological diseases of the present invention can estimate the type of mental and neurological diseases with only the subject's voice. However, in order to further increase the estimation accuracy, elements possessed by subjects other than voice may be used together.

Examples of the elements possessed by subjects include medical examination data, stress questionnaire results, and cognitive function test results.

In Step S107 in Fig. 3, the calculating part 122 acquires the medical examination data of the subject acquired in advance from the second recording device 120C. The medical examination data is data in which health examination results and the like are recorded when the subject inputs voice or before the subject inputs voice. The medical examination data may be acquired in advance in the form of inquiry through the communication terminal 200 or the like. The medical examination data includes items such as an item indicating whether each of a body mass index, a fasting blood glucose, a hemoglobin A1c, a blood pressure, a total cholesterol, an LDL cholesterol and urea nitrogen is higher than a reference value and an item indicating whether an HDL cholesterol is lower than a reference value. Based on the above items, the physical health value of the subject may be calculated as a physical value.

In addition, there are no decisive factors as biomarkers for psychiatric/neurological diseases at this time, but search for biomarkers for each disease is being conducted. Therefore, when these biomarkers are measured, and the measurement results are combined with results obtained by analyzing voice according to the present invention, it is possible to estimate diseases more reliably.

Examples of biomarkers for Alzheimer's dementia include (1) amyloid beta 42(Aβ42) in the cerebrospinal fluid, (2) amyloid PET imaging, (3) total τ proteins or phosphorylated τ proteins in the cerebrospinal fluid, and (4) temporal lobe and parietal lobe atrophy in the fMRI examination.

Examples of biomarkers for Lewy body dementia include (1) reduction in the number of dopamine transporters in a SPECT scan, (2) reduced MIBG accumulation in MIBG myocardial scintigraphy, and (3) REM sleep without a decrease in muscle activity in polysomnography.

Examples of biomarkers for Parkinson's disease include (1) reduction in the number of dopamine transporters in a SPECT scan, (2) reduced MIBG accumulation in MIBG myocardial scintigraphy, and (3) increase in the number of α-synuclein oligomers in the cerebrospinal fluid.

Examples of biomarkers for vascular dementia include lacunar infarction, leukoaraiosis, cerebral hemorrhage, multiple cortical infarction, and other defect lesions in MRI images.

On the other hand, regarding biomarkers for mood disorders such as major depressive disorder, various studies on PEA (ethanolamine phosphate), ribosome genes, and the like have been conducted, but have yet to establish anything.

In addition, the second recording device 120C records stress response data in which the response of the subject to stress questionnaire is described in addition to the health data. The calculating part 122 performs a scoring process for the response data, and calculates a stress value indicating the reception degree of stress influence on the subject based on the response data.

In the stress questionnaire, for example, a scoring process defined according to the questionnaire such as simple work-related stress questionnaire is performed on the response data of the subject, and information about the stress received by the subject is acquired. The information about stress includes, for example, at least one of a scale of the intensity of stress received by the examinee, a scale of subjective symptoms of the examinee, a scale of tolerance of the examinee with respect to stress, a scale of resilience of the examinee from the stress state, and a scale of surrounding support for the examinee.

The scale of the intensity of stress received by the examinee indicates the intensity of stress received by the examinee when the examinee answers questions. For example, the scale of the stress intensity is determined from answers to 17 questions included in the section "A" among a total of 57 questions in the simple work-related stress questionnaire. Here, the scale of the stress intensity may be determined from a combination of a factor for a psychological burden in the workplace in the simple work-related stress questionnaire or the like and a stress factor in general private life. In addition, the scale of the stress intensity may be determined using, for example, the interpersonal stress event scale (Tsuyoshi Hashimoto, "A study of classification of interpersonal stress events in college students," the Japanese Society of Social Psychology, Vol. 13, No.1, pp. 64-75, 1997).

The scale of subjective symptoms of the examinee indicates the psychological state when the examinee is answering, and for example, it is determined from answers to 29 questions included in the section "B" in the simple work-related stress questionnaire. The scale of subjective symptoms is for checking psychological and physical subjective symptoms due to the psychological burden and indicates the state of the result of received stress. Here, the cause of stress that causes subjective symptoms is not limited to work.

In addition, the scale of subjective symptoms may be determined using a scale for measuring the depressed state such as Beck Depression Inventory (BDI: Beck et al., "An Inventory for measuring depression," Arch. Gen. Psychiatry, Vol.4, pp.561-571, 1961), Self-Rating Depression Scale (SDS: Zunk et al., "Self-Rating Depression Scale in an Outpatient Further Validation of the SDS," Arch. Gen. Psychiatry, Vol.13, pp.508-515, 1965), geriatric depression scale (GDS), Self-Rating Questionnaire for Depression (SQR-D), Hamilton Depression Rating Scale (HAM-D), or Patient Health Questionnaire-9 (PHQ-9). These tests are a score-based method, and the scores of each test can be evaluated as normal, borderline, or suspicion of depression. In addition, although it is not possible to distinguish between diseases using only evaluation of depression symptoms, the estimation system of the present invention can assist disease determination.

For example, in BDI, the maximum score is 63 points, 0 to 10 points are in a normal range, 11 to 16 points are slightly neurotic and mildly depressed. 17 to 20 points indicate the boundary of depression and that specialist treatment is necessary. 21 to 30 points indicate moderate depression and that specialist treatment is necessary. 31 to 40 points indicate severe depression and that specialist treatment is necessary. 41 points or more indicate extremely severe depression and that specialist treatment is necessary.

In addition, in PHQ-9, the maximum score is 27 points, and the degree of depression symptoms includes 0 to 4 points for no symptoms, 5 to 9 points for mild symptoms, 10 to 14 points for moderate symptoms, 15 to 19 points for moderate to severe symptoms, and 20 to 27 points for severe symptoms.

A scale of surrounding support for the examinee indicates the status of the cooperation system with people who interact with the examinee such as workplace bosses and colleagues, family, friends, and neighbors. For example, the scale of surrounding support for the examinee is determined from answers of 9 questions included in the section "C" in the simple work-related stress questionnaire. The scale of surrounding support indicates that stress received by the examinee is likely to be relieved and stress tolerance of the examinee will increase as surrounding support increases. Here, the scale of surrounding support may be determined using a social support scale for college students (Katauke Yasushi, Ohnuki Naoko, "Development and Validation of Social Support Scales for Japanese College Students," Rissho University, Annual Report of The Japanese Psychological Association, No.5, pp. 37-46, 2014.

The scale of tolerance of the examinee with respect to stress is a scale indicating whether the examinee easily has mental disorders when received the same degree of stress, and is determined, for example, using some of answers to questions from the section "A" to the section "C" in the simple work-related stress questionnaire. Here, it is preferable to appropriately determine which answers for questions are used to determine the stress tolerance scale. That is, as described in the stress vulnerability model, regarding the stress tolerance scale, some people develop diseases and some people do not develop diseases when they receive the same stress, and if the examinee is more vulnerable to stress (has less tolerance), diseases are highly likely to develop with less stress. Therefore, a second calculating part 260 calculates the stress tolerance of the examinee together with the stress load at the same time, and thus a risk of developing diseases can be determined more accurately.

Here, the stress tolerance is considered to include two elements: vulnerability and resilience, and there is an overlapping part between the two elements. When the stress tolerance scale is determined, one of the two elements may be used, or both of the elements may be used. Since a plurality of risk factors are associated in the process of onset, determining the scale of vulnerability in the stress tolerance scales is investigated in consideration of various risk factors. For example, regarding a scale for vulnerability, a short version of narcissistic vulnerability scale (Yuichiro Uechi, Kazuhiro Miyashita, "Narcissistic vulnerability, self-discrepancy, and self-esteem as predictors of prophensity for social phobia," The Japanese Journal of Personality, Vol. 17, pp. 280-291, 2009) may be exemplified. In addition, as another scale for vulnerability, Japanese-version Rumination-Reflection Questionnaire (Keisuke Takano, Yoshihiko Tanno, "A study of development of Japanese-version Rumination-Reflection Questionnaire," The Japanese Journal of Personality, Vol. 16, pp.259-261, 2008) and Japanese-version Brief Core Schema Scale (Takashi Yamauchi, Anju Sudo, Yoshihiko Tanno, "Reliability and validity of Japanese-version Brief Core Schema Scale," The Japanese Psychological Association, Vol. 79, pp.498-505, 2009) may be exemplified.

In addition, resilience generally means a defense factor against stress in psychology and indicates resistance to stress and an ability to recover from diseases. It is thought that resilience includes innate qualitative resilience and acquired resilience after birth. Examples of resilience scales include a bidimensional resilience factor scale (Mari Hirano, "A study of classification of resilience qualitative factor and acquired factor," The Japanese Journal of Personality, Vol. 19, pp. 94-106, 2010). Here, the qualitative resilience includes four factors: optimism, leadership, sociability, and ability to act, as subscales, and the acquired resilience includes three factors: problem-solving confidence, self-understanding, and understanding the psychology of others, as subscales. Since resilience also differs depending on the examinee, a risk of developing diseases can be determined more accurately by measuring the resilience of the examinee together with the stress load at the same time.

Regarding a cognitive function test, a revised Hasegawa dementia rating scale (HDS-R) and Mini-Mental State Examination (MMSE) are generally used. In addition thereto, Wechsler Memory Scale-Revised (WMS-R), N-type mental function test (N type), the National Mental Institute screening and test (the National Mental Institute), Montreal Cognitive Assessment (MoCA), Dementia Assessment Sheet for Community-based Integrated Care System-21 items (DASC-21), and the like can be used. These tests are a score-based method, and the scores of each test can be evaluated as normal, borderline, mild cognitive impairment, or severe cognitive impairment. In addition, although it is not possible to distinguish between diseases using only evaluation of the cognitive function, the estimation system of the present invention can assist disease determination.

For example, in HDS-R, the maximum score is 30 points, a score of 20 points or less is considered as suspected dementia, its sensitivity is 93% and its specificity is 86%.

In addition, in MMSE, the maximum score is 30 points, a score of 23 points or less is considered as suspected dementia, and its sensitivity is 81% and its specificity is 89%. Thus, a score of 27 points or less is considered as suspected mild cognitive impairment (MCI).

(Estimation part)

The estimation part 123 in Fig. 1 will be described with reference to steps in Fig. 3. In Step S108, the estimation part 123 estimates diseases based on the predictive value of the disease acquired by the calculating part 122 and/or medical examination data. In Step S107, when the calculating part 122 also calculates the stress value, the disease may be estimated based on the predictive value of the disease, the medical examination data, the physical value, and the stress value.

Next, in Step S109, the estimation part 123 determines whether advisory data corresponding to the disease has been selected. The advisory data corresponding to the disease is an advice for preventing the disease or avoiding aggravating of the disease when the subject receives the advisory data. The advisory data created for each disease is recorded in the second recording device 120C. When the advisory data corresponding to the disease has been selected, the process proceeds to Step S111. When the advisory data is not selected, in Step S110, advisory data corresponding to the symptoms of the subject is selected from the second recording device.

Next, in Step S111, the estimation part 123 instructs the estimation result of the disease and the selected advisory data to be transmitted to the first receiving part 131 of the display part 130 through the second transmitting part 124.

Next, in Step S112, the estimation part 123 instructs the output part 132 of the display part 130 to output the estimation result and the advisory data.

Finally, the estimation system 100 ends the estimation process. The estimation system 100 repeatedly performs the processes of Step S101 to Step S112 whenever voice data of the subject is received from the communication terminal 200.

In the above embodiment, the calculating part 122 calculates the predictive value of the disease of the subject based on the feature value F(a) using the voice data of the subject acquired from the input part 110. The estimation part 123 can estimate the health condition or disease of the subject based on the calculated predictive value of the disease of the subject and/or medical examination data recorded in the second recording device 120C, and present the estimation results together with the advisory data to the subject.

As described above, Step S101 to Step S112 shown in Fig. 3 are performed by the estimation system 100, and thus it is possible to provide the estimation system 100, the estimation program and the estimation method through which it is possible to estimate psychiatric/neurological diseases from which the subject is suffering from voice spoken by the subject, prevent aggravation of the diseases, and enable patients to receive appropriate treatment based on accurate determination of the disease.

The estimation system 100 of the present invention is used by doctors, clinical psychologists, nurses, laboratory technicians, counselors and any other person who handles the device of the present invention while they face the subject whose voice is to be acquired. In a room in which a quiet environment is maintained such as a treatment room or a counseling room, the system is used while one or more persons who handle the device of the present invention explain how to directly acquire voice in an open state to the subject whose voice is to be acquired. In addition, the subject whose voice is to be acquired enters an audibility test room or various other test rooms, and the system is used while the above handler sees the subject through the glass or images on the monitor. When the subject is in a remote place such as at home, there is a method in which a voice acquisition method is explained for the subject in advance, and the subject himself or herself performs recording at a designated date and time. When acquisition is performed in a remote place, voice can be acquired while confirming the identity of the person with a camera image using a separate communication line.

In addition, it is possible to acquire voice when the subject goes to a medical institution or a counseling room and perform estimation, and it is possible to acquire voice as one inspection item of health examinations and clinical surveys for companies and local governments and perform estimation.

(Operation of associating plurality of disorders with voice data-voice acquisition)

Procedures when the estimation program is created will be described.

In order to perform an operation of associating a plurality of disorders with voice data, voices of the following patients and healthy subjects were acquired between December 25, 2017 and May 30, 2018.

- Voices of patients with Alzheimer's dementia 20 cases
- Voices of patients with Lewy body dementia 20 cases
- Voices of patients with Parkinson's disease 20 cases
- Voices of patients with major depressive disorder 20 cases
- Voices of patients with bipolar disorder 16 cases
- Voices of patients with atypical depression 19 cases
- Voices of healthy subjects 20 cases

Here, these patients were patients whose diseases were determined according to the criteria of DSM-5 or ICD-10 by doctors in specialized fields such as psychiatry and neurology. In addition, the doctor confirmed that there were no complications of other psychiatric/neurological diseases by performing PHQ-9, MMSE or the like.

It was confirmed that healthy subjects did not have depression symptoms or decline in the cognitive function by performing PHQ-9, MMSE, or the like.

A pin microphone (commercially available from Olympus Corporation) and a portable recorder (commercially available from Roland Corporation) were used for voice acquisition. The voice data was recorded in an SD card.

For the utterance content, within 17 sentences shown in the following Table 1, the subjects read 1 to 13 sentences twice and read 14 to 17 sentences once.

[Table 1]

| | | Read sentence | Intention |
|---|---|---|---|
| | 1 | I-ro-ha-ni-ho-he-to | Strings are familiar and sentences are easily uttered without much emotion |
| | 2 | A- i -u-e-o-ka-ki -ku-ke-ko | |
| | 3 | Today is sunny | |
| | 4 | Once upon a time | |
| | 5 | Galapagos Islands | Sentence including all sounds that use respective parts of the palate (g sound), tongue (I sound), lip (p sound) in generation of consonants |
| | 6 | I'm very fine | Content described as symptoms in DSM-5 |
| | 7 | I slept well yesterday | |
| | 8 | I have an appetite | |
| | 9 | I feel peaceful | |
| | 10 | I'm hot-tempered | |
| | 11 | Tired and exhausted | |
| | 12 | Let's walk upward | Easy-to-read sentences with positive emotion |
| | 13 | I'll do my best | |
| | 14 | Ahhh (continue for 3 seconds or longer) | Long vowel: used to check tone structure |
| | 15 | Ehhh (continue for 3 seconds or longer) | |
| | 16 | Uhhh (continue for 3 seconds or longer) | |
| | 17 | Patakapatakapataka (repeat 5 times or more) | Check articulation (it is said that it is difficult to pronounce La line in the case of cerebral infarction and Ta line in the case of cerebellar infarction) |

When voice was acquired, the subjects heard explanations about use for the study for analyzing the relationship between Voices of patients with psychiatric/neurological diseases and the diseases, and the utterance content and voice acquisition method, and signed a written consent form. In addition, the acquired data including voice was symbolized and managed in a format that cannot identify individuals.

(Creation of estimation program 1)

For each subject 1, long utterances were broken down into two parts and unclear utterances were excluded from a total of 30 utterances including 1 to 13 utterances (26 utterances per case each 2 times) and 14 to 17 utterances (4 utterances per case each time) among the above 17 types of utterance content. Thereby, a total of 4,186 utterances including 613 utterances of patients with Alzheimer's dementia, 573 utterances of patients with Lewy body dementia, 608 utterances of patients with Parkinson's disease, 619 utterances of patients with major depressive disorder, 484 utterances of patients with bipolar disorder, 573 utterances of patients with atypical depression, and 615 utterances of healthy subjects were obtained.

A plurality of acoustic parameters were extracted from these utterances, acoustic parameter features such as the frequency and intensity were associated with the diseases of the subjects, and thus the plurality of acoustic parameters associated with 7 types including the above six diseases and healthy subjects were obtained. For each disease, a plurality of acoustic feature values were extracted by selecting a combination of a plurality of acoustic parameters. In addition, the estimation program 1 was created based on the feature value F(a) for estimating any of the six diseases or healthy subjects by combining them.

Here, the disease estimation program used in the disease estimation system of the present invention does not analyze content of words spoken regarding acoustic feature values. This estimation program extracts acoustic feature values from utterances and calculates predictive values of diseases from the created feature values. Therefore, there is an advantage that the program does not depend on languages. However, when examinees or subjects actually speak, they cannot fluently speak sentences that are not in their native language, and this may affect acoustic characteristics. Therefore, for example, when diseases of examinees whose native language is English are estimated, preferably, first, voices of patients and healthy subjects whose native languages are English are collected and analyzed to create an estimation program for English, and diseases are estimated using English utterances using this program. Similarly, estimation programs for languages other than Japanese and English can be created.

When an estimation program for English is created and diseases are estimated using this program, for example, the following English sentences may be exemplified as sentences read by subjects or examinees.

For example, the following sentences can be exemplified as English sentences.

(1) A, B, C, D, E, F, G
(2) Prevention is better than cure.
(3) Time and tide wait for no man.
(4) Seeing is believing.
(5) A rolling stone gathers no moss.
(6) One, Two, Three, Four, Five, Six, Seven, Eight

For languages, there are no particular limitations on sentences to be spoken, but well-known sentences that anyone can easily read are preferable. In addition, long vowels such as "Ahhh," "Ehhh," and "Uhhh" are preferable because anyone can utter them regardless of their native languages.

A commercially available feature value extraction program can be used as a method of extracting acoustic feature values from the spoken voice. Specific examples thereof include openSMILE.

A large number of acoustic feature values can be extracted from many utterances (1 phrase) using such an acoustic feature value extraction program, but in the present invention, 7,440 acoustic feature values were used as a base.

When each estimation program was created, some acoustic feature values suitable for determining diseases were selected from 7,440 acoustic feature values, and they were combined to set a calculation formula for calculating predictive values of diseases so that each patient can be distinguished for each disease.

Therefore, the number of acoustic feature values and combinations thereof used differ depending on what kind of determination is performed (that is, depending on each estimation program).

(Estimation result based on estimation program 1)

In the estimation program 1, for each of healthy subjects, Lewy body dementia, Alzheimer's dementia, Parkinson's disease, major depressive disorder, bipolar disorder, and atypical depression, acoustic feature values were selected, and predictive values for healthy subjects and for each disease were calculated.

Among 615 utterances spoken by healthy subjects, 383 utterances were estimated to be spoken by healthy subjects, 246 utterances were estimated to be spoken by patients with Lewy body dementia, 72 utterances were estimated to be spoken by patients with Alzheimer's dementia, 51 utterances were estimated to be spoken by patients with Parkinson's disease, 18 utterances were estimated to be spoken by patients with major depressive disorder, 30 utterances were estimated to be spoken by patients with bipolar disorder, and 15 utterances were estimated to be spoken by patients with atypical depression. Therefore, the ratio (sensitivity: recall) of utterances estimated to be spoken by healthy subjects among the utterances of actually healthy subjects was 62.3%.

In addition, among 600 utterances estimated to be spoken by healthy subjects, 383 utterances were actually spoken by healthy subjects, 38 utterances were actually spoken by patients with Lewy body dementia, 67 utterances were spoken by patients with Alzheimer's dementia, 45 utterances were spoken by patients with Parkinson's disease, 32 utterances were spoken by patients with major depressive disorder, 28 utterances were spoken by patients with bipolar disorder, and 7 utterances were spoken by patients with atypical depression. Therefore, the ratio (accuracy: precision) of utterances of actually healthy subjects among the utterances estimated to be spoken by healthy subjects was 63.8%.

In addition, among 940 utterances spoken by patients with major depressive disorder, 46 utterances were estimated to be spoken by healthy subjects, 43 utterances were estimated to be spoken by patients with Lewy body dementia, 24 utterances were estimated to be spoken by patients with Alzheimer's dementia, 15 utterances were estimated to be spoken by patients with Parkinson's disease, 748 utterances were estimated to be spoken by patients with major depressive disorder, 38 utterances were estimated to be spoken by patients with bipolar disorder, and 26 utterances were estimated to be spoken by patients with atypical depression. Therefore, the ratio (sensitivity, recall) of utterances estimated to be spoken by patients with major depressive disorder among the utterances of actual patients with major depressive disorder was 79.6%.

In addition, among 923 utterances estimated to be spoken by patients with major depressive disorder, 748 utterances were actually spoken by patients with major depressive disorder, 29 utterances were actually spoken by patients with Lewy body dementia, 8 utterances were spoken by patients with Alzheimer's dementia, 33 utterances were spoken by patients with Parkinson's disease, 37 utterances were spoken by healthy subjects, 33 utterances were spoken by patients with bipolar disorder, and 35 utterances were spoken by patients with atypical depression. Therefore, the ratio (accuracy, precision) of utterances of actual patients with major depressive disorder among the utterances estimated to be spoken by patients with major depressive disorder was 81.0%.

In addition, among 589 utterances spoken by patients with Lewy body dementia, 41 utterances were estimated to be spoken by healthy subjects, 347 utterances were estimated to be spoken by patients with Lewy body dementia, 67 utterances were estimated to be spoken by patients with Alzheimer's dementia, 61 utterances were estimated to be spoken by patients with Parkinson's disease, 29 utterances were estimated to be spoken by patients with major depressive disorder, 24 utterances were estimated to be spoken by patients with bipolar disorder, and 20 utterances were estimated to be spoken by patients with atypical depression. Therefore, the ratio (sensitivity, recall) of utterances estimated to be spoken by patients with Lewy body dementia among the utterances of actual patients with Lewy body dementia was 58.8%.

In addition, among 602 utterances estimated to be spoken by patients with Lewy body dementia, 347 utterances were actually spoken by patients with Lewy body dementia, 46 utterances were actually spoken by patients with major depressive disorder, 73 utterances were spoken by patients with Alzheimer's dementia, 68 utterances were spoken by patients with Parkinson's disease, 32 utterances were spoken by healthy subjects, 25 utterances were spoken by patients with bipolar disorder, and 14 utterances were spoken by patients with atypical depression. Therefore, the ratio (accuracy, precision) of utterances of actual patients with Lewy body dementia and major depressive disorder among the utterances estimated to be spoken by patients with Lewy body dementia was 57.6%.

Based on these results, for each disease, AUC curves showing the degree of estimation of healthy subjects and other diseases were obtained. As shown in Fig. 5, it can be understood that the degree of estimation of each psychiatric disorder was about 0.8 for all AUCs, and the accuracy was high.

(Creation of estimation program 1-2)

The estimation program 1-2 extracts and estimates major depressive disorder among psychiatric/neurological diseases including at least Alzheimer's dementia, Lewy body dementia, Parkinson's disease, major depressive disorder, atypical depression and bipolar disorder, which have symptoms similar to each other.

The types of utterances used in the analysis are 17 types of utterances (phrases) as in the estimation program 1.

First, for each utterance phrase, 7,440 acoustic feature values were extracted using openSMILE which is software for extracting acoustic feature values. Data that significantly differed depending on facilities in which voice was acquired was excluded, a classification algorithm was created by a method using boosting and decision trees in combination using feature values of 1,547 data items, and predictive values of diseases for distinguishing major depressive disorder and other diseases were generated.

Here, since 2,236 phrases spoken by 5 patients with diseases other than major depressive disorder were used and there were 940 utterances of patients with major depressive disorder, in order to avoid imbalance in the number of data items, pseudo-data of utterances of patients with major depressive disorder was created using a method of Synthetic Minority Over-sampling Technique (SMOTE), and the number of phrases spoken by patients with major depressive disorder was 3,139.

The data was divided into 10 parts, and cross-validation of the classification algorithm of the estimation program 1-2 was performed 10 times.

(Estimation result based on estimation program 1-2)

Table 2 shows the estimation results based on the estimation program 1-2. When major depressive disorder and other psychiatric/neurological diseases were distinguished using the estimation program 1-2, the recall for major depressive disorder was 91.1%, the recall for other psychiatric/neurological diseases was 93.8%, and the accuracy was 92.7%. In addition, the AUC was 0.977.

[Table 2]

| | | Estimation result | | | |
|---|---|---|---|---|---|
| | | Major depressive disorder | Other psychiatric/neurological diseases | Recall | |
| Actual | Major depressive disorder | 2,944 | 195 | 0.911 | |
| | Other psychiatric/neurological diseases | 200 | 2,036 | 0.938 | |
| | Precision | 0.913 | 0.936 | 0.927 | Accuracy |

Other psychiatric/neurological diseases include 5 diseases bipolar disorder, atypical depression, Alzheimer's dementia and Lewy body dementia, and Parkinson's disease.

(Application example of estimation program 1-2/estimation program 1-2-2)

Since the estimation program 1-2-2 can distinguish between major depressive disorder and other psychiatric/neurological diseases, it is possible to distinguish between major depressive disorder and other diseases where symptoms are similar to those of major depressive disorder in actual clinical situations. Specifically, for example, the program can be used to determine whether it is major depressive disorder or bipolar disorder, or determine whether it is major depressive disorder or atypical depression.

(Creation of estimation program 1-3)

The estimation program 1-3 extracts and estimates Lewy body dementia among psychiatric/neurological diseases including at least Alzheimer's dementia, Lewy body dementia, Parkinson's disease, major depressive disorder, atypical depression and bipolar disorder, which have symptoms similar to each other. The types of utterances used in the analysis are 17 types of utterances (phrases) as in the estimation program 1.

First, for each utterance phrase, 7,440 acoustic feature values were extracted using openSMILE which is software for extracting acoustic feature values. Feature values that significantly differed depending on facilities in which voice was acquired were excluded, a classification algorithm was created by a method using boosting and decision trees in combination using 1,547 feature values, and predictive values of diseases for distinguishing Lewy body dementia and other diseases were generated.

Here, since 2,987 phrases spoken by 5 patients with diseases other than Lewy body dementia were used and there were 583 utterances of patients with Lewy body dementia, in order to avoid imbalance in the number of data items, pseudo-data of utterances of patients with Lewy body dementia was created using a method of Synthetic Minority Over-sampling Technique (SMOTE), and the number of phrases spoken by patients with Lewy body dementia was 2,696.

The data was divided into 10 parts, and cross-validation of the classification algorithm of the estimation program 1-3 was performed 10 times.

(Estimation result based on estimation program 1-3)

Table 3 shows the estimation results based on the estimation program 1-3. When Lewy body dementia and other psychiatric/neurological diseases were distinguished using the estimation program 1-3, the recall for Lewy body dementia was 88.6%, the recall for other psychiatric/neurological diseases was 89.8%, and the accuracy was 89.2%. In addition, the AUC was 0.959.

[Table 3]

| | | Estimation result | | | |
|---|---|---|---|---|---|
| | | Lewy body dementia | Other psychiatric/neurological diseases | Recall | |
| Actual | Lewy body dementia | 2,389 | 307 | 0.886 | |
| | Other psychiatric/neurological diseases | 295 | 2,602 | 0.898 | |

(continued)

| | | Estimation result | | | |
|---|---|---|---|---|---|
| | | Lewy body dementia | Other psychiatric/neurological diseases | Recall | |
| | Precision | 0.89 | 0.894 | 0.892 | Accuracy |

Other psychiatric/neurological diseases include 5 diseases major depressive disorder, bipolar disorder, atypical depression, Alzheimer's dementia and Parkinson's disease.

(Application example of estimation program 1-3/estimation program 1-3-2)

Since the estimation program 1-3-2 can distinguish between Lewy body dementia and other psychiatric/neurological diseases, it is possible to distinguish between Lewy body dementia and other diseases where symptoms are similar to those of Lewy body dementia in actual clinical situations. Specifically, for example, the program can be used to determine whether it is Lewy body dementia or Alzheimer's dementia or it is Lewy body dementia or Parkinson's disease.

(Creation of estimation program 2)

In the estimation program 1, first, acoustic parameters associated for each disease were selected, and these acoustic parameters were then combined to generate feature values to create an estimation program. In the estimation program 2, first, two target diseases were selected, a combination of acoustic parameters for distinguishing between these two diseases was created, a program was created to estimate diseases by combining acoustic parameters for distinguishing between two diseases and calculating the superiority or inferiority of the accuracy.

The estimation program 2 selects two of 6 types of diseases including Alzheimer's dementia, Lewy body dementia, Parkinson's disease, bipolar disorder, atypical depression and major depressive disorder, and healthy subjects, estimates which disease that the subject is more likely to have, and performs combining of two diseases and estimates the most probable disease (or wellness).

For example, cases in which it is estimated whether the subject has Alzheimer's dementia or Lewy body dementia, whether the subject has Lewy body dementia or Parkinson's disease, whether the subject has major depressive disorder or bipolar disorder, or whether the subject has these diseases or is healthy will be described. Here, in order to estimate whether the subject has Lewy body dementia or is a healthy subject, for example, parameters A, B, and C may be used. On the other hand, most effective parameters for estimating any combination of two diseases may differ such that parameters D, E, and F are preferably used in order to estimate whether the subject has Alzheimer's dementia or Lewy body dementia.

In this case also, in a combination of two diseases, when the superiority or inferiority of the accuracy is calculated, it is possible to rank the accuracy of 6 types of diseases and healthy subjects. Here, if the difference between the first rank and the second rank is a certain level or larger, it can be determined and estimated that the subject has the first rank disease. In addition, if the difference between the first rank and the second rank is not large, the probability of being suffering from the two diseases can be expressed.

In this manner, it is possible to estimate which of Alzheimer's dementia and Lewy body dementia, which of Lewy body dementia and Parkinson's disease, or which of major depressive disorder and bipolar disorder that the subject has with a high possibility or whether the subject is healthy.

(Creation of estimation program 2-1)

As shown in Table 4, voices of healthy subjects and patients with Alzheimer's dementia were acquired from several hospitals.

For each subject, unclear utterances were excluded from a total of 30 utterances including 1 to 13 utterances (26 utterances per case each 2 times) and 14 to 17 utterances (4 utterances per case each time) among 17 types of utterance content listed in Table 1. Thereby, 737 utterances of patients with Alzheimer's dementia and 1,287 utterances of healthy subjects were acquired.

[Table 4]

| Recording facility | | Male | Female | Total (number of people) | Age Average±standard deviation | Number of utterance (total) | Application |
|---|---|---|---|---|---|---|---|
| Healthy subject | Hospital A | 3 | 4 | 7 | 68.1±9.2 | 190 | Feature selection, learning |
| | Hospital B | 0 | 6 | 6 | 71.9+17.8 | 156 | |
| | Hospital C | 3 | 4 | 7 | 36.7±9.8 | 235 | |
| | Hospital D | 10 | 4 | 14 | 39.8±8.9 | 504 | Feature selection, verification |
| | Hospital E | 12 | 11 | 23 | 53.4±12.9 | 783 | |
| Patients with Alzheimer's dementia | Hospital A | 2 | 8 | 10 | 83.9±3.3 | 254 | Feature selection, learning |
| | Hospital C | 5 | 5 | 10 | 81.3±6.5 | 400 | |
| | Hospital F | 9 | 13 | 22 | 78.9±7.3 | 737 | Feature selection, verification |

For each patient utterance, 7,440 acoustic feature values were extracted using openSMILE which is software for extracting acoustic feature values.

Examples of acoustic feature values include (1) Fourier transform, Mel Frequency Cepstral coefficient, voice probability, zero-crossing rate, fundamental frequency (F0), and the like as feature values similar to physical feature values calculated in parts of frames, (2) a moving average, a time change, and the like as processing in the time direction of the above (1), and (3) average, maximum, minimum, median, quartiles, variance, kurtosis, skewness, and the like as statistical values for the frame in the same file of the above (2).

Among 7,440 acoustic feature values, first, feature values that may depend on the recording environment were excluded and 197 acoustic feature values were selected.

The J48 decision tree algorithm (C4.5 Weka Implementation) was used to distinguish between healthy subjects and patients with Alzheimer's dementia using these 197 acoustic feature values, voices acquired in three hospitals were used for learning, and voices acquired in other three hospitals were used for verification.

(Estimation result based on estimation program 2-1)

Result 1: classification performance for each utterance

An estimation program was used for each utterance to estimate whether the utterance was for patients with Alzheimer's dementia or healthy subjects. The results are shown in Table 5.

[Table 5]

| | | Estimation result | | | |
|---|---|---|---|---|---|
| | | Healthy | Alzheimer's dementia | Recall | |
| Actual | Healthy | 887 | 400 | 0.689 | Specificity |
| | Alzheimer's dementia | 359 | 378 | 0.513 | Sensitivity |
| | Precision | 0.712 | 0.486 | 0.625 | Accuracy |
| | | Negative precision | Positive precision | | |

Result 2: classification performance for each subject

For utterances for each subject (about 30 utterances each), the number of determinations in which the utterances were estimated to be spoken by patients with Alzheimer's dementia or estimated to be spoken by healthy subjects was measured, and the result with the most decisions under majority rule was used as the estimation result according to the estimation program. The results are shown in Table 6.

[Table 6]

| | | Estimation result | | | |
|---|---|---|---|---|---|
| | | Healthy | Alzheimer's dementia | Recall | |
| Actual | Healthy | 35 | 2 | 0.946 | Specificity |
| | Alzheimer's dementia | 9 | 13 | 0.591 | Sensitivity |
| | Precision | 0.796 | 0.867 | 0.814 | Accuracy |
| | | Negative precision | Positive precision | | |

(Creation of estimation program 2-2)

From patients with Parkinson's disease and Lewy body dementia, 20 utterances of "Ah" for 3 seconds each (for 20 people) were obtained. Regarding these utterances, characteristic acoustic parameters such as the frequency and intensity were extracted and characteristics of acoustic parameters were associated with the disease of the subject to obtain various acoustic parameter groups associated with utterances in patients with Parkinson's disease and Lewy body dementia. Characteristic acoustic parameters for distinguishing between Parkinson's disease and Lewy body dementia were selected, and the two selected acoustic parameters were combined to create the estimation program 2-2 based on feature values for estimating whether the subject has Parkinson's disease or Lewy body dementia.
Here, when the estimation program was created, the utterance was divided into 10 parts and cross-validation was performed.

(Estimation result based on estimation program 2-2)

For 20 utterances of "Ah" of patients with Parkinson's disease (for 20 people) and 20 utterances of "Ah" of patients with Lewy body dementia (for 20 people), it was estimated by the estimation program 2-2 whether the subject has Parkinson's disease or Lewy body dementia.
As a result, among 20 utterances of patients with Parkinson's disease, 17 utterances were estimated to be spoken by patients with Parkinson's disease and 3 utterances were estimated to be spoken by patients with Lewy body dementia. In addition, among 20 utterances of patients with Lewy body dementia, 18 utterances were estimated to be spoken by patients with Lewy body dementia and 2 utterances were estimated to be spoken by patients with Parkinson's disease.
Therefore, the accuracy of the estimation program 2-2 was 87.5%. In addition, for Lewy body dementia, the recall was 90% and the precision was 85.7%. In addition, for Parkinson's disease, the recall was 85%, and the precision was 89, 5%.

(Estimation program 2-3)

The estimation program 2-3 that estimates whether the subject has Lewy body dementia or is healthy was created using the same method as in the estimation program 2-1.
For 19 utterances of "Ah" of healthy subjects (for 19 people) and 20 utterances of "Ah" of patients with Lewy body dementia (for 20 people), it was estimated by the estimation program 2-3 whether the subject is healthy or has Lewy body dementia.
As a result, among 19 utterances of healthy subjects, 19 utterances were estimated to be spoken by healthy subjects and 0 utterances were estimated to be spoken by patients with Lewy body dementia. In addition, among 20 utterances of patients with Lewy body dementia, 17 utterances were estimated to be spoken by patients with Lewy body dementia and 3 utterances were estimated to be spoken by healthy subjects.
Therefore, the accuracy of the estimation program 2-3 was 92.3%. In addition, the sensitivity of Lewy body dementia was 85%, the specificity was 100%, the positive precision was 100%, and the negative precision was 86.4%.

(Estimation program 2-4)

The estimation program 2-4 that estimates whether the subject is healthy or has major depressive disorder was created using the same method as in the estimation program 2-1.

For 19 utterances of "Ah" of healthy subjects (for 19 people) and 18 utterances of "Ah" of patients with major depressive disorder (for 18 people), it was estimated by the estimation program 2-4 whether the subject is healthy or has major depressive disorder.

As a result, among 19 utterances of healthy subjects, 17 utterances were estimated to be spoken by healthy subjects and 2 utterances were estimated to be spoken by patients with major depressive disorder. In addition, among 18 utterances of patients with major depressive disorder, 17 utterances were estimated to be spoken by with patients with major depressive disorder and 1 utterance was estimated by spoken by healthy subjects.

Therefore, the accuracy of the estimation program 2-4 was 91.9%.

In addition, the sensitivity of major depressive disorder was 94.4%, the specificity was 89.5%, the positive precision was 89.5%, and the negative precision was 94.4%.

(Estimation program 2-5)

The estimation program 2-5 is a program for distinguishing between healthy subjects and patients with major depressive disorder.

First, for each phrase (about 30 utterances for each person) spoken by 20 healthy subjects and 20 patients with major depressive disorder, 7,440 acoustic feature values were extracted using openSMILE which is software for extracting acoustic feature values. Feature values that significantly differed depending on facilities in which voice was acquired were excluded, and 1,547 feature values were selected. Then, using a method using boosting and decision trees in combination, predictive values of diseases for distinguishing between healthy subjects and major depressive disorder were generated from the feature values to create a classification algorithm. The estimation results for each phrase are shown in Table 7.

In determination of major depressive disorder in the estimation program 2-5, the sensitivity was 81.4%, the specificity was 85.5%, and the accuracy was 83.5%.

[Table 7]

| Estimation program 2-5 | | | | | |
|---|---|---|---|---|---|
| Estimation result in parts of phrase | | | | | |
| | | Estimation result | | | |
| | | Healthy | Major depressive disorder | Recall | |
| Actual | Healthy | 526 | 89 | 0.855 | Specificity |
| | Major depressive disorder | 115 | 504 | 0.814 | Sensitivity |
| | Precision | 0.821 | 0.85 | 0.835 | Accuracy |
| | | Negative precision | Positive precision | | |

Table 8 shows the results summarized for each person who was determined to have major depressive disorder or be healthy for each phrase based on the estimation program 2-5. In addition, a rate of being determined to be healthy for each person was calculated, subjects with a rate of being determined to be healthy of 60% or more were estimated to be healthy, and subjects with a rate of being determined to be healthy of less than 60% were estimated to be patients with major depressive disorder. As a result, as shown in Table 9, all of the 20 healthy subjects were estimated to be healthy, and all of the 20 patients with major depressive disorder were estimated to be patients with major depressive disorder. That is, the specificity, the sensitivity, and the accuracy were all 100%.

[Table 8]

| Subject (healthy people) | Number of times that subject was determined to be healthy | Number of times that subject was determined to have major depressive disorder | Total number of utterances | Rate of being determined to be healthy |
|---|---|---|---|---|
| HE1 | 29 | 1 | 30 | 97% |

(continued)

| Subject (healthy people) | Number of times that subject was determined to be healthy | Number of times that subject was determined to have major depressive disorder | Total number of utterances | Rate of being determined to be healthy |
|---|---|---|---|---|
| HE2 | 32 | 3 | 35 | 91% |
| HE3 | 22 | 8 | 30 | 73% |
| HE4 | 32 | 2 | 34 | 94% |
| HE5 | 28 | 2 | 30 | 93% |
| HE6 | 31 | 0 | 31 | 100% |
| HE7 | 29 | 1 | 30 | 97% |
| HE8 | 29 | 1 | 30 | 97% |
| HE9 | 29 | 2 | 31 | 94% |
| HE10 | 29 | 1 | 30 | 97% |
| HE11 | 25 | 5 | 30 | 83% |
| HE12 | 29 | 1 | 30 | 97% |
| HE13 | 26 | 5 | 31 | 84% |
| HE14 | 24 | 6 | 30 | 80% |
| HE15 | 21 | 9 | 30 | 70% |
| HE16 | 22 | 8 | 30 | 73% |
| HE17 | 20 | 10 | 30 | 67% |
| HE18 | 22 | 8 | 30 | 73% |
| HE19 | 23 | 10 | 33 | 70% |
| HE20 | 24 | 6 | 30 | 80% |

| Subject (patients with major depressive disorder) | Number of times that subject was determined to be healthy | Number of times that subject was determined to have major depressive disorder | Total number of utterances | Rate of being determined to be healthy |
|---|---|---|---|---|
| MDD1 | 13 | 17 | 30 | 43% |
| MDD2 | 9 | 21 | 30 | 30% |
| MDD3 | 17 | 13 | 30 | 57% |
| MDD4 | 5 | 38 | 43 | 12% |
| MDD5 | 10 | 22 | 32 | 31% |
| MDD6 | 9 | 21 | 30 | 30% |
| MDD7 | 1 | 29 | 30 | 3% |
| MDD8 | 0 | 30 | 30 | 0% |

| MDD9 | 3 | 28 | 31 | 10% |
|---|---|---|---|---|
| MDD10 | 1 | 29 | 30 | 3% |
| MDD11 | 1 | 29 | 30 | 3% |
| MDD12 | 8 | 22 | 30 | 27% |

(continued)

| MDD13 | 2 | 31 | 33 | 6% |
| MDD14 | 12 | 18 | 30 | 40% |
| MDD15 | 6 | 24 | 30 | 20% |
| MDD16 | 3 | 27 | 30 | 10% |
| MDD17 | 2 | 28 | 30 | 7% |
| MDD18 | 0 | 30 | 30 | 0% |
| MDD19 | 7 | 23 | 30 | 23% |
| MDD20 | 6 | 24 | 30 | 20% |

[Table 9]

| Estimation program 2-5<br>Estimation result in parts of person | | | | | |
| --- | --- | --- | --- | --- | --- |
| | | Estimat tion result | | | |
| | | Healthy | Major depressive disorder | Recall | |
| Actual | Healthy | 20 | 0 | 1 | Specificity |
| | Major depressive disorder | 0 | 20 | 1 | Sensitivity |
| | Precision | 1 | 1 | 1 | Accuracy |
| | | Negative precision | Positive precision | | |

Therefore, when the estimation program 1-2 and the estimation program 2-5 are used together, it is possible to estimate major depressive disorder with a high determination probability from among patients with Alzheimer's dementia, Lewy body dementia, Parkinson's disease, major depressive disorder, atypical depression and bipolar disorder and healthy subjects.

(Estimation program 2-6)

The estimation program 2-6 is a program for distinguishing between healthy subjects and patients with Lewy body dementia. A classification algorithm was created in the same procedures as in the estimation program 2-5 except that utterance phrases of 20 healthy subjects and 20 patients with Lewy body dementia were used as voices. The estimation results for each phrase are shown in Table 10.

In determination of Lewy body dementia in the estimation program 2-6, the sensitivity was 81.5%, the specificity was 83.1%, and the accuracy was 82.2%.

[Table 10]

| | | Estimation result | | | |
| --- | --- | --- | --- | --- | --- |
| | | Healthy | Lewy body dementia | Recall | |
| Actual | Healthy | 511 | 104 | 0.831 | Specificity |
| | Lewy body dementia | 125 | 549 | 0.815 | Sensitivity |
| | Precision | 0.803 | 0.841 | 0.822 | Accuracy |
| | | Negative precision | Positive precision | | |

Table 11 shows the results summarized for each person who was estimated to have Lewy body dementia or be healthy for each phrase based on the estimation program 2-6. In addition, a rate of being determined to be healthy for each person was calculated, subjects with a rate of being determined to be healthy of 60% or more were estimated to be healthy and subjects with a rate of being determined to be healthy of less than 60% were estimated to be patients with Lewy body dementia. As a result, as shown in Table 12, all of the 20 healthy subjects were estimated to be healthy, and among 20 patients with Lewy body dementia, 19 patients were estimated to be patients with Lewy body dementia and 1 patient was estimated to be healthy. That is, the specificity was 100%, the sensitivity was 95%, and the accuracy was 97.5%.

[Table 11]

| Subject (healthy people) | Number of times that subject was determined to be healthy | Number of times that subject was determined to have Lewy body dementia | Total number of utterances | Rate of being determined to be healthy (%) |
|---|---|---|---|---|
| HE1 | 23 | 7 | 30 | 77% |
| HE2 | 21 | 14 | 35 | 60% |
| HE3 | 24 | 6 | 30 | 80% |
| HE4 | 26 | 8 | 34 | 76% |
| HE5 | 20 | 10 | 30 | 67% |
| HE6 | 28 | 3 | 31 | 90% |
| HE7 | 22 | 8 | 30 | 73% |
| HE8 | 28 | 2 | 30 | 93% |
| HE9 | 31 | 0 | 31 | 100% |
| HE10 | 29 | 1 | 30 | 97% |
| HE11 | 25 | 5 | 30 | 83% |
| HE12 | 23 | 7 | 30 | 77% |
| HE13 | 23 | 8 | 31 | 74% |
| HE14 | 25 | 5 | 30 | 83% |
| HE15 | 24 | 6 | 30 | 80% |
| HE16 | 26 | 4 | 30 | 87% |
| HE17 | 28 | 2 | 30 | 93% |
| HE18 | 29 | 1 | 30 | 97% |
| HE19 | 28 | 5 | 33 | 85% |
| HE20 | 28 | 2 | 30 | 93% |
| Subject (patients with Lewy body dementia) | Number of times that subject was determined to be healthy | Number of times that subject was determined to have Lewy body dementia | Total number of utterances | Rate of being determined to be healthy (%) |
| DLB1 | 3 | 29 | 32 | 9% |
| DLB2 | 1 | 29 | 30 | 3% |
| DLB3 | 2 | 41 | 43 | 5% |
| DLB4 | 5 | 25 | 30 | 17% |
| DLB5 | 7 | 39 | 46 | 15% |
| DLB6 | 14 | 33 | 47 | 30% |
| DLB7 | 5 | 28 | 33 | 15% |
| DLB8 | 12 | 20 | 32 | 38% |
| DLB9 | 18 | 12 | 30 | 60% |

(continued)

| Subject (patients with Lewy body dementia) | Number of times that subject was determined to be healthy | Number of times that subject was determined to have Lewy body dementia | Total number of utterances | Rate of being determined to be healthy (%) |
|---|---|---|---|---|
| DLB10 | 6 | 26 | 32 | 19% |
| DLB11 | 14 | 16 | 30 | 47% |
| DLB12 | 4 | 26 | 30 | 13% |
| DLB13 | 5 | 25 | 30 | 17% |
| DLB14 | 1 | 29 | 30 | 3% |
| DLB15 | 12 | 18 | 30 | 40% |
| DLB16 | 5 | 38 | 43 | 12% |
| DLB17 | 4 | 26 | 30 | 13% |
| DLB18 | 1 | 33 | 34 | 3% |
| DLB19 | 3 | 27 | 30 | 10% |
| DLB20 | 3 | 29 | 32 | 9% |

[Table 12]

| Estimation program 2-6 Estimation result in parts of person | | | | | |
|---|---|---|---|---|---|
| | | Estimation result | | | |
| | | Healthy | Lewy body dementia | Recall | |
| Actual | Healthy | 20 | 0 | 1 | Specificity |
| | Lewy body dementia | 1 | 19 | 0.95 | Sensitivity |
| | Precision | 0.952 | 1 | 0.975 | Accuracy |
| | | Negative precision | Positive precision | | |

Therefore, when the estimation program 1-3 and the estimation program 2-6 are used together, it is possible to estimate Lewy body dementia with a high determination probability from among patients of Alzheimer's dementia, Lewy body dementia, Parkinson's disease, major depressive disorder, atypical depression and bipolar disorder and healthy subjects.

In addition, as in the estimation program 2-5 and the estimation program 2-6, when an estimation program that classifies a specific disease and other 5 diseases and an estimation program that classifies the specific disease and healthy subjects are combined, it is possible to estimate patients with Alzheimer's dementia, Lewy body dementia, Parkinson's disease, major depressive disorder, atypical depression and bipolar disorder and healthy subjects with a high determination probability.

(Estimation program 2-7)

The estimation program 2-7 is a program for estimating whether the subject is healthy, a patient with major depressive disorder or a patient with Lewy body dementia.

A classification algorithm was created in the same procedures as in the estimation program 2-5 except that utterance phrases of 20 healthy subjects, 20 patients with major depressive disorder, and 20 patients with Lewy body dementia were used as voices. The estimation results for each phrase are shown in Table 13.

The specificity was 67.8%, the sensitivity of Lewy body dementia was 70.3%, the sensitivity of major depressive disorder was 69.5%, and the accuracy was 69.2%.

[Table 13]

| | | Estimation result | | | | |
|---|---|---|---|---|---|---|
| | | Healthy | Lewy body dementia | Major depressive disorder | Recall | |
| Actual | Healthy | 417 | 107 | 91 | 0.678 | Specificity |
| | Lewy body dementia | 102 | 474 | 98 | 0.703 | Sensitivity |
| | Major depressive disorder | 107 | 82 | 430 | 0.695 | Sensitivity |
| | Precision | 0.666 | 0.715 | 0.695 | 0.692 | Accuracy |
| | | Negative precision | Precision | Precision | | |

(Creation of estimation program 3)

For utterances of each subject, estimation was performed for each utterance, and a disease (or wellness) that was most frequently estimated was estimated as a disease (or wellness) that the subject was suffering from. For example, in the results obtained by estimating 32 utterances obtained from a subject A, when 4 utterances were spoken by healthy subjects, 16 utterances were spoken by patients with Lewy body dementia, 3 utterances were spoken by patients with Alzheimer's dementia, 3 utterances were spoken by patients with Parkinson's disease, 2 utterances were spoken by patients with major depressive disorder, 2 utterances were spoken by patients with bipolar disorder, and 2 utterances were spoken by patients with atypical depression, the subject A was estimated to have Lewy body dementia that was most frequently estimated.

(Creation of estimation program 4)

For utterances of each subject, estimation was performed for each utterance, and the probability of suffering from the disease (or being healthy) was estimated based on frequency of estimation. In this case, infrequently estimation results can be excluded.
For example, in the results obtained by estimating 32 utterances obtained from the subject A, when 4 utterances were spoken by healthy subjects, 16 utterances were spoken by patients with Lewy body dementia, 3 utterances were spoken by patients with Alzheimer's dementia, 3 utterances were spoken by patients with Parkinson's disease, 2 utterances were spoken by patients with major depressive disorder, 2 utterances were spoken by patients with bipolar disorder, and 2 utterances were spoken by patients with atypical depression, the estimated number of 3 or less was excluded to make 4 utterances of healthy subjects and 16 utterances of patients with Lewy body dementia, and the probability of having Lewy body dementia was estimated as 80%, and the probability of being healthy was estimated as 20%.

(Creation of estimation program 5)

The estimation program 5 can perform estimation even if two specific types of diseases are mild. For example, mild cognitive impairment (MCI) is defined in DSM-5: mild cognitive impairment due to Alzheimer's disease, frontotemporal mild cognitive impairment, mild cognitive impairment with Lewy bodies, vascular mild cognitive impairment, mild cognitive impairment due to Parkinson's disease, and some other mild cognitive impairment. When dementia is mild, characteristic symptoms of the disease are unclear and diagnosis is difficult in many cases.
Therefore, for example, an algorithm for estimating Alzheimer's dementia and Lewy body dementia, an algorithm for estimating Alzheimer's dementia and frontotemporal dementia, and an algorithm for precisely distinguishing between two specific diseases such as Lewy body dementia and Parkinson's disease were created, and accordingly, it is estimated that the subject has any of the diseases in a mild stage.

(Creation of estimation program 6)

It is estimated that the subjects have any of the diseases belonging to these groups such as II. Schizophrenia Spectrum and Other Psychotic Disorders, III. Bipolar and Related Disorders, IV Depressive Disorders, V. Anxiety Disorders, XVIII. Personality Disorders, XVII. Neurocognitive disorders.

An algorithm for estimating such disease groups is preferably used, for example, when screening for any psychiatric disorder by a health examination or when first seeing a general internal medicine or general practitioner.

For example, major depressive disorder and atypical depression as depressive disorder, bipolar disorder as III. Bipolar and Related Disorders, and Alzheimer's dementia and Lewy body dementia as neurocognitive disorders are selected, and characteristic voice parameters are applied to each of these groups, and thus an algorithm for estimating disease groups is created.

(Creation of estimation program 7)

Depending on how feature values are generated, according to which acoustic parameters are selected, it is possible to estimate which of three groups including a dementia group including 3 diseases: Alzheimer's dementia, Lewy body dementia, and Parkinson's disease, a mood disorder group including 3 diseases: major depressive disorder, bipolar disorder, and atypical depression, and a healthy group the disease belong to or estimate whether the subject is healthy. Two diseases may be added to the dementia group for vascular dementia and frontotemporal dementia, and persistent depressive disorder and cyclothymia may be additionally added to the mood disorder group.

In addition, depending on how feature values are generated, according to which acoustic parameters are selected, it is possible to estimate which of four groups including a group including Alzheimer's dementia and Lewy body dementia, a Parkinson's disease group, a mood disorder group including major depressive disorder, bipolar disorder and atypical depression, and a healthy group the disease belongs to or estimate whether the subject is healthy.

For each subject, long utterances were broken down into two parts and unclear utterances were excluded from a total of 30 utterances including 1 to 13 utterances (26 utterances per case each 2 times) and 14 to 17 utterances (4 utterances per case each time) among the above 17 types of utterance content. Thereby, a total of 4,183 utterances including 2,356 utterances of patients with Alzheimer's dementia, Lewy body dementia, and Parkinson's disease (these are a dementia group), 1,342 utterances of patients with major depressive disorder, bipolar disorder, and atypical depression (there are a mood disorder group), and 585 utterances of healthy subjects (healthy subject group) were obtained.

Characteristic acoustic parameters were extracted from these utterances, characteristics of acoustic parameters such as the frequency and intensity were associated with the disease of the subject to obtain various acoustic parameter groups associated with three types: the dementia group, the mood disorder group and healthy subjects. Characteristic acoustic parameters for each disease were selected, and additionally the selected acoustic parameters were combined to create an estimation program 7 based on feature values for distinguishing whether the subject belongs to a dementia group or a mood disorder group or is healthy.

Here, when the estimation program was created, the utterances of each group were divided into 5 parts and cross-validation was performed 5 times.

(Estimation result based on estimation program 7)

Among 585 utterances spoken by healthy subjects, 364 utterances were estimated to be spoken by healthy subjects, 180 utterances were estimated to be spoken by patients in the dementia group, and 41 utterances were estimated to be spoken by patients in the mood disorder group. Therefore, the ratio (sensitivity: recall) of utterances estimated to be spoken by healthy subjects among the utterances of actually healthy subjects was 62.2%.

In addition, among 558 utterances estimated to be spoken by healthy subjects, 364 utterances were spoken by actually healthy subjects, 148 utterances were actually spoken by patients in the dementia group, and 46 utterances were actually spoken by patients in the mood disorder group. Therefore, the ratio (accuracy:precision) of utterances of actually healthy subjects among the utterances estimated to be spoken by healthy subjects was 65.2%.

In addition, among 2,356 utterances spoken by patients in the dementia group, 148 utterances were estimated to be spoken by healthy subjects, 2,126 utterances were estimated to be spoken by patients in the dementia group, and 82 utterances were estimated to be spoken by patients in the mood disorder group. Therefore, the ratio (sensitivity: recall) of utterances estimated to be spoken by patients in the dementia group among the utterances of patients in the actual dementia group was 90.2%.

In addition, among 2,389 utterances estimated to be spoken by patients in the dementia group, 2,126 utterances were estimated to be spoken by patients in the actual dementia group, 180 utterances were estimated to be spoken by actually healthy subjects, and 83 utterances were estimated to be spoken by actual patients in the mood disorder group. Therefore, the ratio (accuracy:precision) of utterances of patients in the actually dementia group among the utterances estimated to be spoken by patients in the dementia group was 89.0%.

In addition, among 1,342 utterances spoken by patients in the mood disorder group, 46 utterances were estimated to be spoken by healthy subjects, 83 utterances were estimated to be spoken by patients in the dementia group, and 1,213 utterances were estimated to be spoken by patients with mood disorders. Therefore, the ratio (sensitivity:

EP 3 821 815 A1

recall) of utterances estimated to be spoken by patients in the mood disorder group among the utterances of actual patients in the mood disorder group was 90.4%.

In addition, among 1,336 utterances estimated to be spoken by patients in the mood disorder group, 1,213 utterances were spoken by patients in the actual mood disorder group, 41 utterances were spoken by actually healthy subjects, and 82 utterances were spoken by patients in the actual dementia group. Therefore, the ratio (accuracy:precision) of utterances of patients in the actually mood disorder group among the utterances estimated to be spoken by patients in the mood disorder group was 90.8%.

The overall accuracy was 86.5%.

According to this estimation algorithm, it is possible to estimate a group of the disease that the subject has.

Like the estimation program 6, an algorithm for estimating a disease group such as the estimation program 7 is preferably used when screening for any psychiatric disorder by a health examination or when first seeing a general internal medicine or general practitioner. Then, after the disease group is estimated, the above estimation program 2, an estimation program 8 to be described below, or the like are used for each disease group, and thus it is possible to estimate a specific disease among mental and neurological diseases.

(Creation of estimation program 8)

Depending on how feature values are generated, according to which acoustic parameters are selected, it is possible to estimate whether the disease belongs to one specific disease or another disease in paired comparison such as whether it is Alzheimer's dementia or another disease and whether it is Lewy body dementia or other diseases among mental and neurological diseases.

For example, when voice data of Lewy body dementia and voices of Alzheimer's dementia and Parkinson's disease in combination as other diseases are analyzed and acoustic parameters that can distinguish them are selected, it is possible to create a program for estimating Lewy body dementia from the dementia group. Then, if voice data of an estimation target subject is calculated by this estimation program, it is possible to estimate whether the subject has Lewy body dementia or other dementia.

In addition, for example, when voice data of major depressive disorder and voices of Lewy body dementia, Alzheimer's dementia, Parkinson's disease, atypical depression, and bipolar disorder in combination as other diseases are analyzed and acoustic parameters that can distinguish them are selected, it is possible to create a program for estimating major depressive disorder from the mental-neurological diseases. Then, if voice data of an estimation target subject is calculated by this estimation program, it is possible to estimate whether the subject has major depressive disorder or other diseases.

(Creation of estimation program 9)

As in the estimation program 1, depending on how feature values are generated, according to which acoustic parameters are selected, it is possible to distinguish between Alzheimer's dementia, Lewy body dementia, Parkinson's disease, major depressive disorder, bipolar disorder, atypical depression, and healthy subjects. Alternatively, after six diseases and healthy subjects are distinguished once, three diseases Alzheimer's dementia, Lewy body dementia, and Parkinson's disease are combined as a dementia group, and three diseases major depressive disorder, bipolar disorder, and atypical depression are combined as a mood disorder group, and as a result, it is possible to estimate whether the disease is in the dementia group or the mood disorder group or whether the subject is healthy.

(Creation of estimation program 10)

As in the estimation program 9, it is estimated whether the disease is in the dementia group or the mood disorder group or whether the subject is healthy. Alternatively, regarding a plurality of sentences for each person, the disease is assigned to each sentence, and the disease to which the largest number of sentences is assigned is then estimated to be the disease of the subject, and it is estimated whether the disease is in the dementia group or the mood disorder group or whether the subject is healthy.

Voices of 15 patients with Alzheimer's dementia, 12 patients with bipolar disorder, 14 patients with atypical depression, 15 patients with Lewy body dementia, 15 patients with major depressive disorder, 15 patients with Parkinson's disease, and 15 healthy subjects were used as learning data.

Voices of 5 patients with Alzheimer's dementia, 4 patients with bipolar disorder, 5 patients with atypical depression, 5 patients with Lewy body dementia, 5 patients with major depressive disorder, 5 patients with Parkinson's disease, and 5 healthy subjects were used as test data.

Here, the learning data and the test data were randomly distributed.

For voices used for learning data, patients and healthy subjects uttered the above 17 sentences (twice each

30

for Nos. 1 to 13, and once each for Nos. 14 to 17) and 30 utterances for each person were obtained.

Characteristic acoustic parameters were extracted from these utterances, characteristics of acoustic parameters such as the frequency and the intensity were associated with the disease of the subject to obtain various acoustic parameter groups associated with 7 types: the above six diseases and the healthy subjects.

Characteristic acoustic parameters for each disease were selected, and additionally the selected acoustic parameters were combined, and thus it was estimated whether the subject had any of six diseases or was healthy for each utterance sentence of each person, and subsequently, the estimation program 10 was created based on feature values for estimating whether the disease was in the dementia group or the mood disorder group, and whether the subject was healthy.

(Estimation result based on estimation program 10)

Regarding test data, among 17 sentences in Table 1, 8 phrases of 2 "A-i-u-e-o-ka-ki-ku-ke-ko," 3 "Today is sunny," 6 "I'm very fine," 8 "I have an appetite," 9 "I feel peaceful," 10 "I'm hot-tempered," 12 "Let's walk upward," and 13 "I'll do my best" (a total of 16 sentences, 2 times each) were used. For each utterance, it was estimated whether the subject has any of the diseases or is healthy. Among the estimated diseases, diseases estimated to be three diseases Alzheimer's dementia, Lewy body dementia, and Parkinson's disease were determined as a dementia group disease. In addition, diseases estimated to be three diseases major depressive disorder, bipolar disorder, and atypical depression were determined as a mood disorder group disease.

Here, the numbers of 16 utterances for each person that were determined to have a dementia group disease, a mood disorder group disease, or to be healthy were summed and the result with the largest number of determinations was used as a final determination result.

(Result 1) Patients with Alzheimer's dementia (patient code AD03):

13 utterances were determined to indicate a dementia group disease, 1 utterance was determined to indicate a mood disorder group disease, and 2 utterances were determined to indicate wellness. Therefore, it was finally estimated as a dementia group disease. This was the same for the dementia group to which the actual disease Alzheimer's dementia belongs.

Table 14 shows the results for 34 people determined in the same manner.

[Table 14]

| Patient code | Disease name | Disease group | Number of determinations (for each group) | | | Determination | Correctness |
|---|---|---|---|---|---|---|---|
| | | | Cognitive disease group | Mood disorder group | Healthy | | |
| AD02 | AD | C | 12 | 0 | 4 | C | O |
| AD03 | AD | C | 13 | 1 | 2 | C | O |
| AD07 | AD | C | 13 | 0 | 3 | C | O |
| AD11 | AD | C | 14 | 0 | 2 | C | O |
| AD15 | AD | C | 9 | 1 | 6 | C | O |
| BP11 | BP | M | 0 | 16 | 0 | M | O |
| BP12 | BP | M | 10 | 4 | 2 | C | × |
| BP14 | BP | M | 0 | 7 | 9 | H | × |
| BP16 | BP | M | 0 | 10 | 6 | M | O |
| DAF02 | DAF | M | 1 | 12 | 3 | M | O |
| DAF06 | DAF | M | 0 | 16 | 0 | M | O |
| DAF08 | DAF | M | 0 | 16 | 0 | M | O |
| DAF14 | DAF | M | 1 | 12 | 3 | M | O |
| DAF19 | DAF | M | 1 | 14 | 1 | M | O |

(continued)

| Patient code | Disease name | Disease group | Number of determinations (for each group) | | | Determination | Correctness |
|---|---|---|---|---|---|---|---|
| | | | Cognitive disease group | Mood disorder group | Healthy | | |
| DLB01 | DLB | C | 9 | 0 | 7 | C | O |
| DLB02K | DLB | C | 14 | 0 | 2 | C | O |
| DLB02M | DLB | C | 12 | 4 | 0 | C | O |
| DLB04 | DLB | C | 12 | 1 | 3 | C | O |
| DLB06 | DLB | C | 14 | 0 | 2 | C | O |
| HE01 | HE | H | 5 | 0 | 11 | H | O |
| HE02 | HE | H | 0 | 7 | 9 | H | O |
| HE03H | HE | H | 11 | 0 | 5 | C | × |
| HE03K | HE | H | 10 | 0 | 6 | C | × |
| HE09 | HE | H | 3 | 13 | 0 | M | × |
| MD05 | MDD | M | 0 | 12 | 4 | M | O |
| MD12 | MDD | M | 6 | 10 | 0 | M | O |
| MD16 | MDD | M | 3 | 13 | 0 | M | O |
| MD17 | MDD | M | 0 | 15 | 1 | M | O |
| MD19 | MDD | M | 3 | 12 | 1 | M | O |
| PD02 | PD | C | 14 | 0 | 2 | C | O |
| PD03 | PD | C | 13 | 0 | 3 | C | O |
| PD05H | PD | C | 13 | 0 | 3 | C | O |
| PD05K | PD | C | 12 | 0 | 4 | C | O |
| PD07 | PD | C | 10 | 0 | 6 | C | O |
| AD: Alzheimer's dementia<br>BP: bipolar disorder<br>DAF: atypical depression<br>DLB: Lewy body dementia<br>HE: Healthy<br>MDD: Major depressive disorder<br>PD: Parkinson's disease<br>C: disease in cognitive impairment group<br>M: disease in mood disorder group<br>H: healthy | | | | | | | |

The accuracy of the estimation program 10 was 85.3%.

(Creation of estimation program 11)

Which of the diseases the subject has is expressed as a probability, and it is estimated that he subject has a disease shown with the highest probability.

Voices of 20 patients with Lewy body dementia, 20 patients with major depressive disorder, and 20 healthy subjects were used for both learning and testing. As the voice, the long vowel "Ahhh" in item 14 in Table 1 was used.

The acoustic feature values were narrowed down from 7,440 to the top 13 acoustic feature values with a high correlation by logistic regression analysis, and used to calculate the predictive value of the Lewy body dementia disease, the predictive value of the major depressive disorder disease, and the predictive value of the healthy subjects so that the total thereof was 1.

(Estimation result based on estimation program 11)

Table 15 shows subjects that were estimated to have Lewy body dementia or major depressive disorder or to be healthy from the voice of each person. Here, the predictive value of the disease in the table is described as a "mental value."

[Table 15]

|  | Disease (or healthy) | Mental value | | | Determination | Correctness |
|---|---|---|---|---|---|---|
|  |  | DLB | MDD | HE |  |  |
| 1 | DLB | 0.352 | 0.648 | 0 | MDD | × |
| 2 | DLB | 0.582 | 0.125 | 0.293 | DLB | O |
| 3 | DLB | 0.899 | 0.101 | 0 | DLB | O |
| 4 | DLB | 0.65 | 0.348 | 0.002 | DLB | O |
| 5 | DLB | 0.682 | 0.193 | 0.125 | DLB | O |
| 6 | DLB | 0.918 | 0.071 | 0.012 | DLB | O |
| 7 | DLB | 0.743 | 0.212 | 0.045 | DLB | O |
| 8 | DLB | 0.734 | 0.266 | 0 | DLB | O |
| 9 | DLB | 0.739 | 0.186 | 0.075 | DLB | O |
| 10 | DLB | 0.481 | 0.075 | 0.444 | DLB | O |
| 11 | DLB | 0.324 | 0.562 | 0.114 | MDD | × |
| 12 | DLB | 0.329 | 0.33 | 0.341 | HE | × |
| 13 | DLB | 0.853 | 0.147 | 0 | DLB | O |
| 14 | DLB | 0.269 | 0.669 | 0.062 | MDD | × |
| 15 | DLB | 0.206 | 0.543 | 0.251 | MDD | × |
| 16 | DLB | 0.492 | 0.34 | 0.168 | DLB | O |
| 17 | DLB | 0.982 | 0.018 | 0 | DLB | O |
| 18 | DLB | 0.518 | 0.363 | 0.118 | DLB | O |
| 19 | DLB | 0.987 | 0.013 | 0 | DLB | O |
| 20 | DLB | 0.337 | 0.356 | 0.308 | MDD | × |
| 21 | MDD | 0.082 | 0.865 | 0.053 | MDD | O |
| 22 | MDD | 0.379 | 0.491 | 0.13 | MDD | O |
| 23 | MDD | 0.886 | 0.114 | 0 | DLB | × |
| 24 | MDD | 0 | 0.952 | 0.048 | MDD | O |
| 25 | MDD | 0.242 | 0.758 | 0 | MDD | O |
| 26 | MDD | 0.691 | 0.309 | 0 | DLB | × |
| 27 | MDD | 0.351 | 0.268 | 0.381 | HE | × |
| 28 | MDD | 0.25 | 0.75 | 0 | MDD | O |
| 29 | MDD | 0.001 | 0.999 | 0 | MDD | O |
| 30 | MDD | 0.197 | 0.451 | 0.352 | MDD | O |
| 31 | MDD | 0.243 | 0.757 | 0 | MDD | O |

(continued)

|  | Disease (or healthy) | Mental value | | | Determination | Correctness |
|---|---|---|---|---|---|---|
|  |  | DLB | MDD | HE |  |  |
| 32 | MDD | 0.092 | 0.908 | 0 | MDD | O |
| 33 | MDD | 0.447 | 0.151 | 0.402 | DLB | × |
| 34 | MDD | 0.119 | 0.88 | 0.001 | MDD | O |
| 35 | MDD | 0.003 | 0.997 | 0 | MDD | O |
| 36 | MDD | 0.109 | 0.891 | 0 | MDD | O |
| 37 | MDD | 0.015 | 0.031 | 0.954 | HE | × |
| 38 | MDD | 0.14 | 0.288 | 0.572 | HE | × |
| 39 | MDD | 0.392 | 0.608 | 0 | MDD | O |
| 40 | MDD | 0.451 | 0.521 | 0.028 | MDD | O |
| 41 | HE | 0.03 | 0.141 | 0.829 | HE | O |
| 42 | HE | 0.277 | 0.125 | 0.598 | HE | O |
| 43 | HE | 0.223 | 0.131 | 0.645 | HE | O |
| 44 | HE | 0.211 | 0.319 | 0.47 | HE | O |
| 45 | HE | 0 | 0 | 1 | HE | O |
| 46 | HE | 0.615 | 0.321 | 0.064 | DLB | × |
| 47 | HE | 0.076 | 0.338 | 0.585 | HE | O |
| 48 | HE | 0.001 | 0 | 0.999 | HE | O |
| 49 | HE | 0 | 0.16 | 0.84 | HE | O |
| 50 | HE | 0.174 | 0.131 | 0.695 | HE | O |
| 51 | HE | 0 | 0 | 1 | HE | O |
| 52 | HE | 0.165 | 0.034 | 0.801 | HE | O |
| 53 | HE | 0 | 0.005 | 0.994 | HE | O |
| 54 | HE | 0.006 | 0 | 0.994 | HE | O |
| 55 | HE | 0.489 | 0.147 | 0.364 | DLB | × |
| 56 | HE | 0.272 | 0.342 | 0.386 | HE | O |
| 57 | HE | 0.043 | 0.042 | 0.915 | HE | O |
| 58 | HE | 0 | 0 | 1 | HE | O |
| 59 | HE | 0.251 | 0.151 | 0.598 | HE | O |
| 60 | HE | 0.001 | 0.055 | 0.943 | HE | O |
| DLB: Lewy body dementia<br>MDD: major depressive disorder<br>HE: healthy | | | | | | |

The accuracy of the estimation program 11 was 76.7%.

Hereinafter, disease groups in DSM (diagnostic and statistical manual of mental disorders) published by American Psychiatric Association will be shown. Here, the latest version is DSM-5 in 2018.

I. Neurodevelopmental Disorders
II. Schizophrenia Spectrum and Other Psychotic Disorders
III. Bipolar and Related Disorders

IV. Depressive Disorders
V. Anxiety Disorders
VI. Obsessive Compulsive and Related Disorders
VII. Trauma and Stressor Related Disorders
VIII. Dissociative Disorders
IX. Somatic Symptom and Related Disorders
X. Feeding and Eating Disorders
XI. Elimination Disorders
XII. Sleep-Wake Disorders
XIII. Sexual Dysfunctions
XIV. Gender Dysphoria
XV. Disruptive, Impulse-Control, and Conduct Disorders
XVI. Substance-Related and Addictive Disorders
XVII. Neurocognitive Disorders
XVIII. Personality Disorders
XIX. Paraphilic Disorders

Hereinafter, diagnostic criteria for major depressive disorder and Lewy body dementia in DSM-5 will be described below. As described above, in the psychiatric disorder, it is still difficult to observe lesions directly or through some objective indexes and the disorder should be determined by symptoms.

1. Major Depressive Disorder

A. 5 (or more) of the following symptoms have been present during the same 2-week period and represent a change from previous functioning. At least one of these symptoms is either (1) depressed mood or (2) loss of interest or pleasure. Note: Do not include symptoms that are clearly attributable to another medical condition.

(1) Depressed most of the day, nearly every day as indicated by subjective report (e.g., feels sad, empty, hopeless) or observation made by others (e.g., appears tearful) Note: In children and adolescents, can be irritable mood.
(2) Markedly diminished interest or pleasure in all, or almost all, activities most of the day, nearly every day (as indicated by subjective account or observation)
(3) Significant weight loss when not dieting or weight gain (e.g., a change of more than 5% of body weight in a month), or decrease or increase in appetite nearly every day. Note: In children, consider failure to make expected weight gain.
(4) Insomnia or hypersomnia nearly every day.
(5) Psychomotor agitation or retardation nearly every day (observable by others, not merely subjective feelings of restlessness or being slowed down
(6) Fatigue or loss of energy nearly every day
(7) Feelings of worthlessness or excessive or inappropriate guilt (which may be delusional) nearly every day (not merely self-reproach or guilt about being sick).
(8) Diminished ability to think or concentrate, or indecisiveness, nearly every day (either by subjective account or as observed by others).
(9) Recurrent thoughts of death (not just fear of dying), recurrent suicidal ideation without a specific plan, or a suicide attempt or a specific plan for committing suicide.

B. The symptoms cause clinically significant distress or impairment in social, occupational, or other important areas of functioning.
C. The episode is not attributable to the physiological effects of a substance or to another medical condition.
Note: Criteria A-C represent a major depressive episode.
Note: Responses to a significant loss (e.g., bereavement, financial ruin, losses from a natural disaster, a serious medical illness or disability) may include the feelings of intense sadness, rumination about the loss, insomnia, poor appetite, and weight loss noted in Criterion A, which may resemble a major depressive episode. Although such symptoms may be understandable or considered appropriate to the loss, the presence of a major depressive episode in addition to the normal response to a significant loss should also be carefully considered. This decision inevitably requires the exercise of clinical judgment based on the individual's history and the cultural norms for the expression of distress in the context of loss.
D. The occurrence of the major depressive episode is not better explained by schizoaffective disorder, schizophrenia, schizophreniform disorder, delusional disorder, or other specified and unspecified schizophrenia spectrum and other psychotic disorders
E. There has never been a manic episode or a hypomanic episode.

2. Lewy body dementia (Major Neurocognitive Disorder with Lewy Bodies)

A. The criteria are met for major or mild neurocognitive disorder.
B. The disorder has an insidious onset and gradual progression.
C. The disorder meets a combination of Core features and Suggestive features for either probable or possible neurocognitive disorder with Lewy bodies

For probable major or mild neurocognitive disorder with Lewy bodies, the individual has 2 core features, or 1 suggestive feature with 1 or more core features
For possible major or mild neurocognitive disorder with Lewy bodies, the individual has only 1 core feature, or 1 or more suggestive features.

(1) Core features

(a) Fluctuating cognition with pronounced variations in attention and alertness
(b) Recurrent visual hallucinations that are well formed and detailed
(c) Spontaneous features of parkinsonism, with onset subsequent to the development of cognitive decline

(2) Suggestive features

(a) Meets criteria for rapid eye movement sleep behavior disorder
(b) Severe neuroleptic sensitivity

D. The disturbance is not better explained by cerebrovascular disease, another neurodegenerative disease, the effects of a substance, or another mental, neurological, or systemic disorder

(Seventh embodiment)

Next, one embodiment in which the zero crossing rate and the Hurst exponent are selected as the second acoustic parameter will be described in detail. Here, the same parts as in the first embodiment will not be described.
The calculating part 122 calculates a zero crossing rate as a degree of intensity in change of the waveform in voice. In addition, the calculating part 122 calculates a Hurst exponent indicating a correlation of changes in the waveform of the voice. The calculating part 122 outputs the calculated zero crossing rate and Hurst exponent for the subject to the estimation part 123.
In order for the estimation part 123 to estimate the predictive value of the disease of the subject (may be described as a mental value) from the zero crossing rate and the Hurst exponent for the subject, the calculating part 122 sets a health reference range indicating a healthy state without suffering from a disease such as depression.
For example, the calculating part 122 reads voice data of a plurality of people whose health conditions indicating whether he or she has a disease such as depression are known from a recording device of the estimation system 100, and calculates a second acoustic parameter including the zero crossing rate and the Hurst exponent of each of the plurality of people from the read voice data.
In addition, the calculating part 122 performs a linear classification process such as linear discriminant analysis and logistic regression analysis in a 2D space of the zero crossing rate and the Hurst exponent with respect to the distribution of the zero crossing rate and the Hurst exponent for the plurality of people calculated by the calculating part 122, and creates feature values based on these linear models.
Next, the calculating part 122 sets a boundary line that separates a region for people suffering from depression or the like from a reference range for healthy people not suffering from depression or the like based on the feature values. A distance from the reference range including the determined boundary line to the score of the zero crossing rate and the Hurst exponent for the subject is calculated as a predictive value of the disease and is output to the estimation part 123.

The estimation part 123 estimates a disease from the predictive value of the disease in the subject. Then, the estimation part 123 outputs information indicating the estimated health condition to the communication terminal 200.

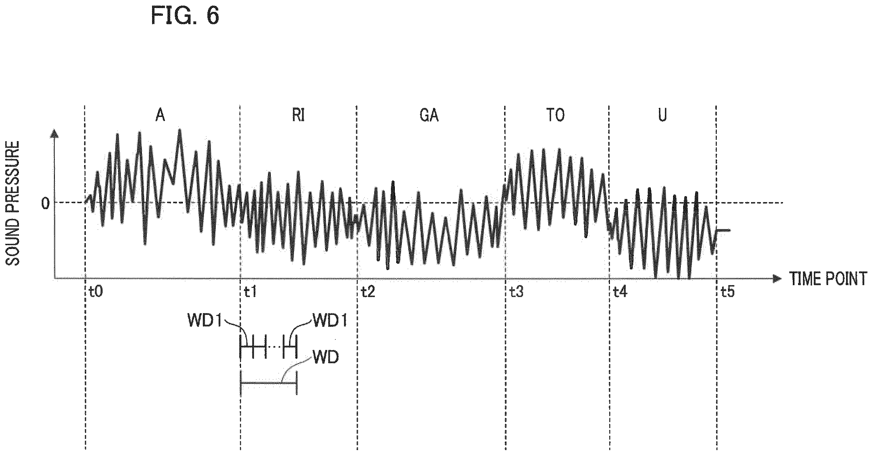

Fig. 6 shows an example of voice data acquired through the communication terminal 200 shown in Fig. 2. Fig. 6 shows change in the sound pressure of the voice spoken by the subject over time, which is acquired through the communication terminal 200. In Fig. 6, the horizontal axis represents time point t and the vertical axis represents sound pressure.

Fig. 6 shows data of the utterance part in which "Arigatou" is spoken within voice data of the utterances spoken by the subject. Time points t0, t1, t2, t3, and t4 indicate start time points at which the sounds "a," "ri," "ga," "to," and "u" included in the utterance part are spoken. Here, a calculation process of the calculating part 122 performed on voice data of the spoken word "Ri" within the utterance part of "Arigatou" will be described. However, the calculating part 122 also performs the same or similar calculation process for other words in "Arigatou" and other utterance parts.

The calculating part 122 calculates the zero crossing rate and the Hurst exponent for each window WD with a number of samples such as 512 using the voice data acquired from the communication terminal 200. As shown in Fig. 6, since the sound pressure largely changes in the utterance of each word, for example, in order to calculate the zero crossing rate, the calculating part 122 calculates an average value of sound pressures for each window WD1 with a number of samples such as 30 smaller than that of the window WD, and sets the average value calculated in each window WD1 as a reference pressure of each window WD1. The calculating part 122 measures the number of times that the sound pressure of the subject crosses the calculated reference pressure (average value) in each window WD1 and calculates the zero crossing rate.

The calculating part 122 calculates the average value of the zero crossing rates calculated in each window WD1 as the zero crossing rate ZCR of the window WD.

On the other hand, the standard deviation $\sigma(\tau)$ of the difference between the sound pressure x(t) at the time point t and the sound pressure x(t+$\tau$) away from the time point t by the time $\tau$ is related as shown in Formula (1). In addition, it is known that there is a power law relationship between the time interval $\tau$ and the standard deviation $\sigma(\tau)$ as shown in Formula (2). Then, in Formula (2), H is the Hurst exponent.

[Math. 2]

$$\sigma(\tau) = \sqrt{\left\langle \left( x(t+\tau) - x(t) - \langle x(t+\tau) - x(t) \rangle \right)^2 \right\rangle} \quad \cdots (1)$$

$$\sigma(\tau) \propto \tau^{H} \quad \cdots (2)$$

For example, in the case of voice data such as white noise, since there is no temporal correlation between data items of voice data, the Hurst exponent H is "0." In addition, as the voice data changes from white noise to pink noise or Brownian noise, that is, as the waveform of the voice has a temporal correlation, the Hurst exponent H indicates a value larger than "0."

For example, when the voice data is Brownian noise, the Hurst exponent H is 0.5. In addition, as the voice data has a stronger correlation than Brownian noise, that is, as the voice data becomes more dependent on the past state, the Hurst exponent H indicates a value between 0.5 and 1.

For example, in the window WD, the calculating part 122 determines a standard deviation $\sigma(\tau)$ of voice data for each $\tau$ at the time interval t between 1 to 15, and performs regression analysis on the determined standard deviation $\sigma(\tau)$ for each time interval $\tau$ to calculate the Hurst exponent H.

The calculating part 122 moves the window WD at predetermined intervals such as a quarter of the width of the window WD, and calculates the zero crossing rate ZCR and the Hurst exponent H in each window WD. Then, the calculating part 122 averages the calculated zero crossing rates ZCR and Hurst exponents H in all of the windows WD, and outputs the averaged zero crossing rate ZCR and Hurst exponent H as the zero crossing rate and the Hurst exponent for the subject to the estimation part 123.

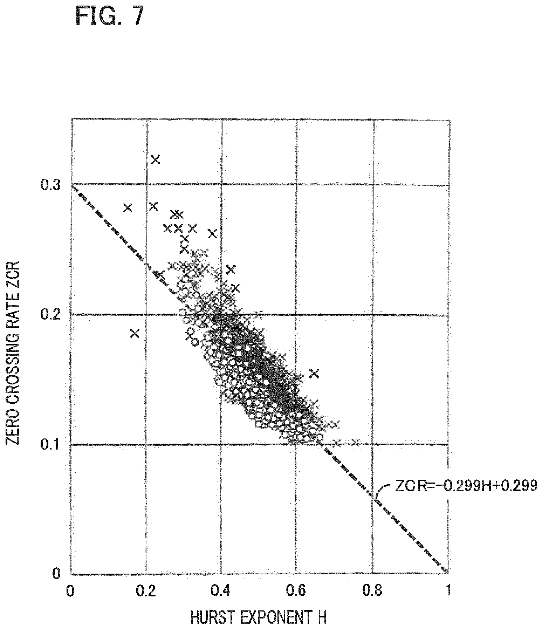

Fig. 7 is an example of the distribution of the zero crossing rate ZCR and the Hurst exponent H for a plurality of people calculated by the calculating part 122 as shown in Fig. 1. In Fig. 7, the vertical axis represents zero crossing rate ZCR and the horizontal axis represents Hurst exponent H.

In addition, in Fig. 7, the zero crossing rate ZCR and the Hurst exponent H for people suffering from a disease such as depression are marked with a cross and the zero crossing rate ZCR and the Hurst exponent H for healthy people are marked with a circle. Here, the distribution of the zero crossing rate ZCR and the Hurst exponent H shown in Fig. 7 is generated using voice data of a total of 1,218 people. Then, among a total of 1,218 people, a total of 697 people are suffering from a disease such as depression and a total of 521 people are healthy.

The calculating part 122 performs a linear classification process such as linear discriminant analysis and logistic regression analysis on the distribution of the zero crossing rate ZCR and the Hurst exponent H for a plurality of people shown in Fig. 7. The calculating part 122 determines a boundary line shown by a dashed line that separates people suffering from a disease such as depression from healthy people.

In Fig. 7, the boundary line shown by the dashed line is represented by ZCR=-0.299H+0.299. The calculating part 122 sets a region below the boundary line shown by the dashed line as a reference range, outputs information about the reference range including the determined boundary line to the estimation part 123, and sets the reference range for the estimation part 123.

Here, in Fig. 7, the vertical axis of the zero crossing rate ZCR and the horizontal axis of the Hurst exponent H are linear axes, but when the boundary line shown by the dashed line is represented by an exponential function or a power function, a logarithmic axis is preferable in order to show the boundary line as a straight line.

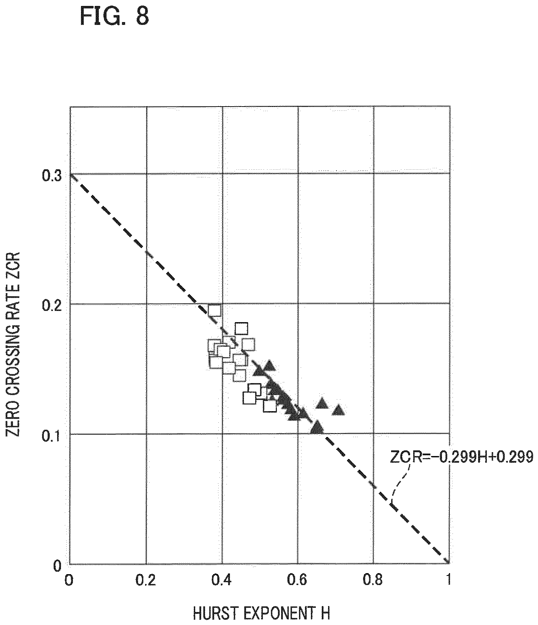

Fig. 8 shows an example of the distribution of the zero crossing rate ZCR and the Hurst exponent H according to a voice data acquisition environment. Like Fig. 7, in Fig. 8, the vertical axis represents zero crossing rate ZCR and the horizontal axis represents Hurst exponent H. In addition, in Fig. 8, the boundary line determined from the distribution of the zero crossing rate ZCR and the Hurst exponent H shown in Fig. 7 by the calculating part 122 is shown by a dashed line.

Fig. 8 shows, for example, the distribution of the zero crossing rate ZCR and the Hurst exponent H calculated using voice data obtained by the communication terminal 200 by sampling the subject's voice at a sampling frequency of 11 kHz with black triangles.

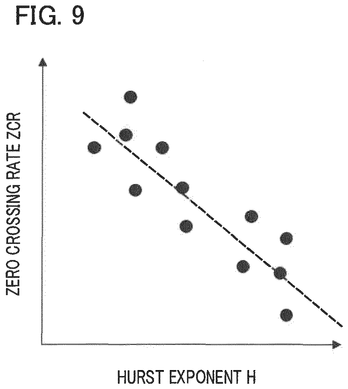

Fig. 9 shows an example of the distribution of the zero crossing rate ZCR and the Hurst exponent H calculated in each window WD using voice data of one person. In Fig. 9, the vertical axis represents zero crossing rate ZCR and the horizontal axis represents Hurst exponent H.

For the distribution of the zero crossing rate ZCR and the Hurst exponent H shown in Fig. 9, a least squares method using a linear function ZCR=$\gamma$H+$\varepsilon$ shown by the dashed line is performed and the slope $\gamma$ and the intercept $\varepsilon$ are calculated. For example, a threshold value that indicates the boundary classifying between the voice of a person suffering from depression and voice of a healthy person is determined as a healthy region using the slope $\gamma$ and the intercept $\varepsilon$ calculated for each of the plurality of people.

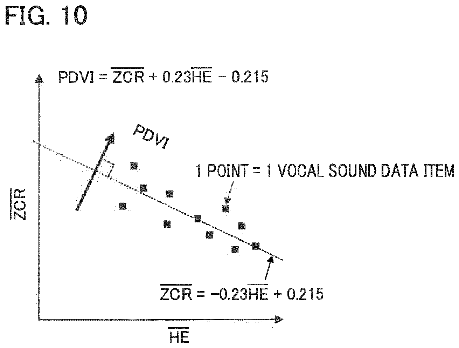

In addition, the zero crossing rate ZCR and the Hurst exponent H calculated in each window WD using voice data of one person are calculated, and average values HE and $\overline{ZCR}$ are obtained from HE and ZCR data in all section. For example, using the $\overline{HE}$ and $\overline{ZCR}$ calculated for each of the plurality of people, a Parkinson's disease index PD-VI=Z$\ddot{e}$R+aHE-$\emptyset$ for classifying voice of a person suffering from Parkinson's disease and voice of a healthy person is determined. In the case of Fig. 10, $\alpha$=0.23, and $\beta$=0.215.

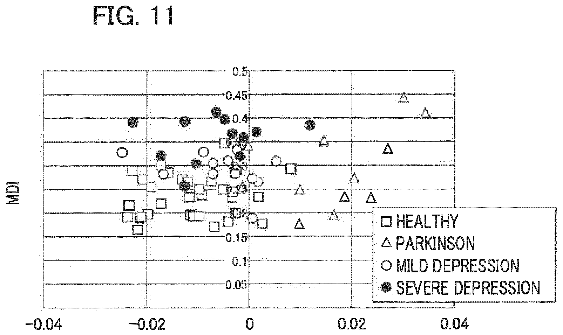

Here, Fig. 11 shows plotted voice data of healthy subjects, patients with depression (mild), patients with depression (severe), and patients with Parkinson's disease using the intercept $\varepsilon$ and PDVI.

On the other hand, for example, the communication terminal 200 down-samples the voice data of the subject sampled at 11 kHz at a sampling frequency of 8 kHz in order to transmit voice data via the network NW to the estimation system 100. Fig. 8 shows the distribution of the zero crossing rate ZCR and the Hurst exponent H calculated using voice data down sampled to 8 kHz with white rectangles.

As shown in Fig. 8, the zero crossing rate ZCR and the Hurst exponent H of the subject are affected by deterioration (increased noise) of sound quality due to down sampling. That is, the zero crossing rate ZCR of the down-sampled voice data shows a larger value than the zero crossing rate ZCR of voice data sampled at 11 kHz because noise increases and the number of times that the sound pressure of voice crosses the reference pressure increases.

On the other hand, the Hurst exponent H of the down-sampled voice shows a smaller value than the Hurst exponent H of voice data sampled at 11 kHz because voice data becomes white noise as noise increases.

However, the zero crossing rate ZCR and the Hurst exponent H are affected by down sampling, and they do not change independently from each other but they change in relation to each other. That is, as shown in Fig. 8, the zero crossing rate ZCR and the Hurst exponent H change along the boundary line shown by the dashed line while having mutual correlation with respect to deterioration of sound quality due to down sampling or the like.

Therefore, deterioration of sound quality due to down sampling or the like does not affect the operation of the estimation part 123 that determines whether the zero crossing rate ZCR and the Hurst exponent H of the subject is included in the reference range. That is, the zero crossing rate ZCR and the Hurst exponent H have robustness against deterioration of sound quality due to down sampling or the like. Then, the estimation system 100 can estimate the health condition of the subject more accurately than in the related art regardless of the voice data acquisition environment.

Here, the estimation system 100 may be applied to, for example, robots, artificial intelligence and automobiles or mobile terminal device applications and services such as call centers, the Internet, smartphones and tablet terminals, and search systems. In addition, the estimation system 100 may be applied to a diagnostic device, an automatic inquiry device, a disaster triage and the like.

The above detailed description will clarify features and advantages of the embodiments. This is intended that the scope of the claims extends to features and advantages of the embodiment examples as described above without departing from the spirit and scope of rights. In addition, those skilled in the art can easily make some improvements and modifications. Therefore, there is no intention to limit the scope of the inventive embodiments to those described above, and it is possible to perform appropriate improvements and equivalents included in the scope disclosed in the embodiments.

[Industrial Applicability]

It is possible to provide an estimation system, an estimation program and an estimation method through which voice spoken by a subject is estimated, a disease from which the subject is suffering is distinguished and estimated, aggravation of the disease is prevented, and patients are able to receive appropriate treatment based on accurate distinguishing of the disease.

[Reference Signs List]

**[0311]**

　　122 Calculating part
　　123 Estimation part
　　100 Estimation system
　　200 Communication terminal

**Claims**

1. An estimation system for psychiatric/neurological diseases, comprising
at least one voice input part including an acquisition part configured to acquire voice data of a subject and a first transmitting part configured to transmit the voice data to a server;
a result display part including a first receiving part configured to receive data of the result estimated in the server and an output part configured to display the data; and
a server including a second receiving part configured to receive the voice data from the first transmitting part, a calculating part configured to calculate a predictive value of a disease based on a combination of at least one acoustic feature value extracted from the voice data of the subject, an estimation part configured to estimate the disease of the subject, and a second transmitting part configured to transmit the estimated data of the result to the display part,
wherein the server includes an arithmetic processing device for executing an estimation program and a recording device in which the estimation program is recorded, and
wherein by the estimation program, the estimation part estimates the disease of the subject selected from the group consisting of a plurality of diseases using the predictive value of the disease as an input.

2. The estimation system according to claim 1,
wherein the plurality of diseases include Alzheimer's dementia, Lewy body dementia, Parkinson's disease, bipolar disorder, atypical depression and major depressive disorder.

3. The estimation system according to claim 1,
wherein the predictive value of the disease inputted into the estimation part is calculated by extracting and combining one or more acoustic feature values that are able to distinguish the disease from any two diseases selected from the group consisting of Alzheimer's dementia, Lewy body dementia, Parkinson's disease, bipolar disorder, atypical depression and major depressive disorder.

4. The estimation system according to claim 3,
wherein the two diseases are a combination of Alzheimer's dementia and Lewy body dementia, a combination of Lewy body dementia and Parkinson's disease, a combination of major depressive disorder and bipolar disorder, or a combination of major depressive disorder and atypical depression.

5. The estimation system according to claim 1,

wherein the predictive value of the disease inputted into the estimation part is calculated by extracting and combining one or more acoustic feature values that are able to distinguish the disease from any one or more diseases selected from the group consisting of Alzheimer's dementia, Lewy body dementia, Parkinson's disease, bipolar disorder, atypical depression and major depressive disorder or the subject is healthy.

6. The estimation system according to claim 5,
   wherein the one disease is major depressive disorder or Lewy body dementia.

7. The estimation system according to claim 1,
   wherein the predictive value of the disease inputted into the estimation part is calculated by extracting and combining one or more acoustic feature values that are able to distinguish the disease from any one disease selected from the group consisting of Alzheimer's dementia, Lewy body dementia, Parkinson's disease, bipolar disorder, atypical depression and major depressive disorder or any other disease.

8. The estimation system according to claim 7,
   wherein the one disease is major depressive disorder or Lewy body dementia.

9. The estimation system according to claim 1,
   wherein the predictive value of the disease inputted into the estimation part is calculated by extracting and combining one or more acoustic feature values that are able to distinguish one or more diseases selected from the group consisting of Alzheimer's dementia, Lewy body dementia, Parkinson's disease, bipolar disorder, atypical depression and major depressive disorder.

10. The estimation system according to claim 1,
    wherein the predictive value of the disease inputted into the estimation part is calculated stepwise, and
    wherein in a first step, one or more acoustic feature values that are able to distinguish between a first group including Alzheimer's dementia, Lewy body dementia, and Parkinson's disease and a second group including major depressive disorder, bipolar disorder and atypical depression are extracted and combined to calculate the predictive value.

11. The estimation system according to claim 10,
    wherein the predictive value of the disease input to the estimation part is calculated stepwise, and wherein in a second step,
    one or more acoustic feature values that are able to distinguish Alzheimer's dementia, Lewy body dementia, and Parkinson's disease are extracted and combined to calculate the predictive value.

12. The estimation system according to claim 10,
    wherein the predictive value of the disease input to the estimation part is calculated stepwise, and wherein in a second step,
    one or more acoustic feature values that are able to distinguish major depressive disorder, bipolar disorder, and atypical depression are extracted and combine to calculate the predictive value.

13. The estimation system for psychiatric/neurological diseases according to any one of claims 1 to 12,
    wherein a subject terminal is provided, the subject terminal includes a display part, and voice data is acquired for each displayed sentence.

14. The estimation system for psychiatric/neurological diseases according to any one of claims 1 to 13,
    wherein the communication terminal displays a psychology test and/or a cognitive function test, and the subject responds.

15. The estimation system for psychiatric/neurological diseases according to claim 1,
    wherein a communication terminal includes the input part and the display part, and is connected to the server via a network.

16. The estimation system for psychiatric/neurological diseases according to claim 1,
    wherein the second recording device additionally records an advisory data collection able to be shown to the subject based on the estimated result, and
    the estimation program enables, based on the result, the estimation part to select advisory data from the advisory data collection of the second recording device and the second transmitting part to transmit the selected data together with the result data to the display part.

17. The estimation system for psychiatric/neurological diseases according to claim 1,
wherein the second recording device records response data containing the responses of the subject to a stress questionnaire, and additionally calculates a value of stress from the response data of the subject, and
wherein the estimation part estimates the disease of the subject based on the predictive value of the disease of the subject and the value of the stress.

18. The estimation system for psychiatric/neurological diseases according to claim 1,
wherein the second recording device records medical examination data of the subject and additionally calculates a physical value from the medical examination data of the subject, and
wherein the estimation part estimates the disease of the subject based on the predictive value of the disease of the subject and the physical value.

19. The estimation system for psychiatric/neurological diseases according to claim 1,
wherein the second recording device records response data containing the responses of the subject to a cognitive function test and additionally calculates a cognitive score from the response data of the subject, and
wherein the estimation part estimates the disease of the subject based on the predictive value of the disease of the subject and the cognitive score.

20. A recording medium in which a program stored in the estimation system according to claim 1 is recorded.

FIG. 1

FIG. 2

INTERFERENCE SYSTEM 100

COMMUNICATION TERMINAL 200

INPUT UNIT 110

ACQUISITION UNIT 111

FIRST TRANSMITTING UNIT 112

DISPLAY UNIT 130

FIRST RECEIVING UNIT 131

OUTPUT UNIT 132

NETWORK NW

SERVER 120

ARITHMETIC PROCESSING DEVICE 120A

SECOND RECEIVING UNIT 121

CALCULATING UNIT 122

INTERFERENCE UNIT 123

SECOND TRANSMITTING UNIT 124

FIRST RECORDING DEVICE 120B

SECOND RECORDING DEVICE 120C

EP 3 821 815 A1

## FIG. 3

START

S101
IS VOCAL SOUND DATA ACQUIRED? — YES

NO

S102
DISPLAY FIXED PHRASE TO OUTPUT UNIT

S103
ACQUIRE VOCAL SOUND DATA FROM INPUT UNIT

S104
TRANSMIT AND RECEIVE VOCAL SOUND DATA BETWEEN INPUT UNIT AND SERVER

S105
IS MENTAL VALUE OF SUBJECT CALCULATED? — YES

NO

S106
CALCULATE MENTAL VALUE BASED ON VOCAL SOUND DATA AND INTERFERENCE PROGRAM

S107
ACQUIRE MEDICAL EXAMINATION DATA OF SUBJECT FROM SECOND RECORDING DEVICE

S108
INFER DISEASE BASED ON METAL VALUE AND MEDICAL EXAMINATION DATA

S109
IS ADVISORY CORRESPONDING TO DISEASE SELECTED? — YES

NO

S110
SELECT ADVISORY CORRESPONDING TO SYMPTOMS OF SUBJECT FROM SECOND RECORDING DEVICE

S111
TRANSMIT AND RECEIVE INTERFERENCE RESULT OF DISEASE AND SELECTED ADVISORY DATA BETWEEN SERVER AND DISPLAY UNIT

S112
OUTPUT INTERFERENCE RESULT AND ADVISORY FROM OUTPUT UNIT

END

FIG. 4A

FEATURE VALUE

FEATURE VALUE

FIG. 4B

## FIG. 5B

FIG. 6

## FIG. 7

FIG. 8

ZCR=−0.299H+0.299

FIG. 9

# FIG. 10

$PDVI = \overline{ZCR} + 0.23\overline{HE} - 0.215$

PDVI

1 POINT = 1 VOCAL SOUND DATA ITEM

$\overline{ZCR} = -0.23\overline{HE} + 0.215$

$\overline{ZCR}$

$\overline{HE}$

FIG. 11

## INTERNATIONAL SEARCH REPORT

| | International application No. |
|---|---|
| | PCT/JP2019/027607 |

**A. CLASSIFICATION OF SUBJECT MATTER**
Int.Cl. A61B10/00(2006.01)i, G10L25/66(2013.01)i

According to International Patent Classification (IPC) or to both national classification and IPC

**B. FIELDS SEARCHED**

Minimum documentation searched (classification system followed by classification symbols)
Int.Cl. A61B10/00, A61B5/16, G10L25/66

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 6312014 B1 (PANASONIC IP MANAGEMENT CO., LTD.) | 1–5,7,9,13–16, |
| | 18 April 2018, paragraphs [0022]–[0055], [0070]– | 18–20 |
| Y | [0071], [0105]–[0106], [0108], [0110]–[0111], fig. | 2–9,17 |
| A | 1–4, 10 & JP 2019–40143 A & WO 2019/044255 A1 & TW | 10–12 |
| | 201913648 A | |
| | | |
| X | JP 2011–255106 A (NAGOYA INSTITUTE OF TECHNOLOGY) | 1–5,7,9,13,15, |
| | 22 December 2011, paragraphs [0006]–[0023], | 20 |
| Y | [0026]–[0049], [0101]–[0104], [0110]–[0111], fig. | 2–9,17 |
| A | 1, 4, 12 (Family: none) | 10–12 |

☒ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 September 2019 (26.09.2019) | 08 October 2019 (08.10.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

## INTERNATIONAL SEARCH REPORT

| International application No. |
| --- |
| PCT/JP2019/027607 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y<br>A | 大下淳一＝日経デジタルヘルス， 「スマホで話す」だけで健康が見える， 日経デジタルヘルス, 16 December 2015, https://tech.nikkeibp.co.jp/dm/atcl/feature/15/327441/121400121/?ST=ndh_print, non-official translation (OSHITA, Junichi= NIKKEI DIGITAL HEALTH, "You can see your health just by 'talking on your smartphone', NIKKEI DIGITAL HEALTH) | 2-9<br>10-12 |
| Y<br>A | 宗未来 他3名， "声"だけで、うつ病はどこまで診断可能か？ 〜 音声感情認識技術にアンサンブル型機械学習モデルを応用したうつ病スクリーニング機能に関する精度の検証, RIETI Discussion Paper Series 16-J-054, September 2016, non-official translation (SO, Mirai et al., "How far can depression be diagnosed with just 'voice'?, Verification of accuracy of depression screening function in which ensemble-type machine learning model is applied to speech emotion recognition technology") | 2-9<br>10-12 |
| Y<br>A | JP 2017-532082 A (SRI INTERNATIONAL) 02 November 2017, abstract, claim 2, paragraphs [0004], [0039] & US 2018/0214061 A1, abstract, paragraphs [0004], [0046] & WO 2016/028495 A1 & EP 3160334 A1 | 2-9<br>10-12 |
| Y<br>A | US 2015/0112232 A1 (MASSACHUSETTS INSTITUTE OF TECHNOLOGY) 23 April 2015, abstract, paragraph [0028] & WO 2015/102733 A1 & EP 3057493 A1 & AU 2014374349 A & CA 2928005 A1 | 2-9<br>10-12 |
| Y<br>A | JP 2017-196314 A (LIFE SCIENCE INSTITUTE, INC.) 02 November 2017, claims 1-13, paragraphs [0002]-[0008], [0102]-[0103] (Family: none) | 17<br>10-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018133356 A
- JP 2019057353 A
- WO 2013069645 A
- JP 2007296169 A
- WO 2006132159 A

**Non-patent literature cited in the description**

- **TSUYOSHI HASHIMOTO.** A study of classification of interpersonal stress events in college students. *the Japanese Society of Social Psychology,* 1997, vol. 13 (1), 64-75
- **BECK et al.** An Inventory for measuring depression. *Arch. Gen. Psychiatry,* 1961, vol. 4, 561-571
- **ZUNK et al.** Self-Rating Depression Scale in an Outpatient Further Validation of the SDS. *Arch. Gen. Psychiatry,* 1965, vol. 13, 508-515
- **KATAUKE YASUSHI ; OHNUKI NAOKO.** Development and Validation of Social Support Scales for Japanese College Students. Rissho University, Annual Report of The Japanese Psychological Association, 2014, 37-46
- **YUICHIRO UECHI ; KAZUHIRO MIYASHITA.** Narcissistic vulnerability, self-discrepancy, and self-esteem as predictors of prophensity for social phobia. *The Japanese Journal of Personality,* 2009, vol. 17, 280-291
- **KEISUKE TAKANO ; YOSHIHIKO TANNO.** A study of development of Japanese-version Rumination-Reflection Questionnaire. *The Japanese Journal of Personality,* 2008, vol. 16, 259-261
- **TAKASHI YAMAUCHI ; ANJU SUDO ; YOSHIHIKO TANNO.** Reliability and validity of Japanese-version Brief Core Schema Scale. The Japanese Psychological Association, 2009, vol. 79, 498-505
- **MARI HIRANO.** A study of classification of resilience qualitative factor and acquired factor. *The Japanese Journal of Personality,* 2010, vol. 19, 94-106